(19) **Europäisches Patentamt / European Patent Office / Office européen des brevets**

(11) **EP 4 595 947 A1**

(12) **EUROPEAN PATENT APPLICATION**
published in accordance with Art. 153(4) EPC

(43) Date of publication:
06.08.2025 Bulletin 2025/32

(21) Application number: 23869847.6

(22) Date of filing: 30.06.2023

(51) International Patent Classification (IPC):
*A61K 9/00* (2006.01)    *A61K 36/884* (2006.01)
*A61K 9/14* (2006.01)    *A61K 9/16* (2006.01)

(52) Cooperative Patent Classification (CPC):
Y02A 50/30

(86) International application number:
PCT/CN2023/104721

(87) International publication number:
WO 2024/066599 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.09.2022 CN 202211174012

(71) Applicant: Dong-e E-jiao Co., Ltd.
Liaocheng, Shandong 252200 (CN)

(72) Inventors:
• QI, Xiaodan
  Liaocheng, Shandong 252200 (CN)
• ZHANG, Yan
  Liaocheng, Shandong 252200 (CN)
• WANG, Zhongchao
  Liaocheng, Shandong 252200 (CN)
• LIU, Haibin
  Liaocheng, Shandong 252200 (CN)
• WANG, Yantao
  Liaocheng, Shandong 252200 (CN)
• ZHANG, Yan
  Liaocheng, Shandong 252200 (CN)
• YANG, Haiju
  Liaocheng, Shandong 252200 (CN)
• KONG, Lingmei
  Liaocheng, Shandong 252200 (CN)
• WANG, Chunyan
  Liaocheng, Shandong 252200 (CN)
• QIAN, Liyan
  Liaocheng, Shandong 252200 (CN)
• ZHAO, Haiqing
  Liaocheng, Shandong 252200 (CN)
• MA, Liping
  Liaocheng, Shandong 252200 (CN)
• HE, Wenhui
  Liaocheng, Shandong 252200 (CN)
• LI, Shidong
  Liaocheng, Shandong 252200 (CN)

(74) Representative: Plougmann Vingtoft a/s
Strandvejen 70
2900 Hellerup (DK)

(54) **PREPARATION METHOD FOR POLYPORUS UMBELLATUS SOUP, POLYPORUS UMBELLATUS EXTRACTUM, AND SOLID FORMULATION OF POLYPORUS UMBELLATUS**

(57) The present invention relates to the technical field of medicines, in particular to a preparation method for a polyporus umbellatus soup, a polyporus umbellatus extractum, and a solid formulation of polyporus umbellatus. The preparation method provided by the present invention comprises: soaking four traditional Chinese medicines in water to give a pre-soaking solution, wherein the four traditional Chinese medicines include polyporus umbellatus, poria cocos, rhizoma alismatis, and talc; performing a first boiling extraction on the pre-soaking solution to give a first water extract and a medicine residue; mixing the medicine residue and water for a second boiling extraction to give a second water extract; performing a first concentration on the first water extract to give a first concentrated solution; combining the first concentrated solution and the second water extract, and performing a second concentration to give a second concentrated solution; and mixing the second concentrated solution with an aqueous solution of donkey-hide gelatin to give the polyporus umbellatus soup. The preparation method for the polyporus umbellatus soup provided by the present invention comprehensively considers water extraction and concentration, which guarantees the consistency of the extraction effect of the ob-

EP 4 595 947 A1

tained polyporus umbellatus soup and a reference sample of a traditional polyporus umbellatus decoction. The contents of effective active ingredients in the obtained polyporus umbellatus soup is high, and the efficacy is good.

```
  ┌──────────┐  ┌──────┐  ┌────────┐  ┌──────┐        ┌────────┐
  │Polyporus │  │Poria │  │Rhizoma │  │Talcum│        │ Asini  │
  │umbellatus│  │cocos │  │Alismatis│ │      │        │ Corii  │
  └──────────┘  └──────┘  └────────┘  └──────┘        │ Colla  │
                                                      └────────┘
                  Adding 8 times to 14
                  times of water
                ┌─────────────┐
                │   Soaking   │
                └─────────────┘
┌──────────────┐ ┌──────────────────────┐
│First aqueous │←│First boiling extraction│
│extract solution│└──────────────────────┘
└──────────────┘    Medicinal residue, adding 6
                    times to 12 times of water
┌──────────────┐ ┌──────────────────┐
│Centrifugation│ │ Second boiling   │
│and fine      │ │   extraction     │
│filtration    │ └──────────────────┘
└──────────────┘ ┌──────────────────┐
┌──────────────┐ │Centrifugation and│
│Concentration │ │fine filtration   │
│of a first    │ └──────────────────┘
│filtrate      │ ┌──────────────────┐
└──────────────┘ │ Second filtrate  │
                 └──────────────────┘
                 ┌──────────────────┐
                 │  Mixing and      │
                 │  concentration   │
                 └──────────────────┘
                   Adjuvant, 6 times to 8 times
                   of water for dissolution  ┌────────────┐
                 ┌──────────────────┐        │Asini Corii │
                 │  Mixing and      │←───────│   Colla    │
                 │  concentration   │        │  aqueous   │
                 └──────────────────┘        │  solution  │
                 ┌──────────────────┐        └────────────┘
                 │  Vacuum drying   │
                 └──────────────────┘
                 ┌──────────────────┐
                 │  Dry granulation │
                 └──────────────────┘
                 ┌──────────────────┐
                 │     Packing      │
                 └──────────────────┘
```

**FIG. 1**

**Description**

The present application claims priority to the Chinese Patent Application CN202211174012.7 filed with the China National Intellectual Property Administration (CNIPA) on September 26, 2022 and entitled "PREPARATION METHOD FOR POLYPORUS UMBELLATUS SOUP, POLYPORUS UMBELLATUS EXTRACTUM, AND SOLID FORMULATION OF POLYPORUS UMBELLATUS", which is incorporated herein by reference in its entirety.

**TECHNICAL FIELD**

The present disclosure relates to the technical field of medicine, and in particular to preparation methods of a Zhu Ling Tang extract solution, a Zhu Ling Tang extract paste, and a Zhu Ling Tang solid preparation.

**BACKGROUND**

Zhu Ling Tang is the 9th formula listed in the *Catalogue of Ancient Classical Formulas* (first batch) issued by the National Administration of Traditional Chinese Medicine. Zhu Ling Tang originates from the *Treatise on Febrile Diseases* (Zhang Zhongjing, Eastern Han Dynasty). Zhu Ling Tang is designed by Zhang Zhongjing to address the pathogenesis of yin deficiency and water-heat interaction. As a basic formula with clinically proven efficacy, recognized by physicians throughout history, Zhu Ling Tang exemplifies the therapeutic approach of nourishing yin and promoting diuresis, holding significant value in modern applications. Under the *Classification of Traditional Chinese medicine Registration and Requirements for Traditional Chinese medicine Application materials,* Zhu Ling Tang is classified as Category 3.1 of New Drugs of traditional Chinese medicine: compound Traditional Chinese Medicine preparations managed according to the *Catalogue of Ancient Classical Formulas.*

Currently, no granular preparation for the ancient classical formula Zhu Ling Tang on is commercially available in China. In compliance with the relevant regulatory requirements specified in the "Regulations on Simplified Registration and Approval of Traditional Chinese Medicine Compound Preparations as Ancient Classical Formulas" and the "Provisions for Drug Registration", the development of modern Zhu Ling Tang preparations should be as follows: based on a Zhu Ling Tang reference sample, Zhu Ling Tang granules are prepared as a finished product via modern pharmaceutical equipment and processes, including extraction, purification, concentration, drying, granulation, and packaging.

Currently, the commercial production of Chinese patent drugs generally adopts water extraction or alcohol extraction, concentration, alcohol precipitation, high-temperature oven-drying, pulverization, molding, and other related technological procedures. This conventional production process offers advantages such as simple equipment, low cost, and technological maturity, making it highly favored in industrial-scale production. However, when applied to the production of classical formulas, this conventional production process faces two significant defects: 1. Ancient classical formulas require the extraction method and solvent consistent with traditional decoction, making alcohol precipitation clearly unsuitable. 2. The high-temperature drying process may degrade thermally unstable components. The above two problems may alter the material basis of classical formula preparations during industrial production, causing them to fail the requirements of the reference sample. Therefore, the current extraction process for Chinese patent drugs may introduce druggability defects in commercializing ancient classical formulas, as it could not guarantee inherent quality consistency between industrial-scale products and those produced by traditional processes.

**SUMMARY**

In view of this, an object of the present disclosure is to provide methods for preparing a Zhu Ling Tang extract solution, a Zhu Ling Tang extract paste, and a Zhu Ling Tang solid preparation. In the present disclosure, the Zhu Ling Tang solid preparation exhibits the basically consistent quality standard with a Zhu Ling Tang reference sample, and exhibits excellent solubility and high bioavailability. The Zhu Ling Tang solid preparation is more convenient to take and carry and exhibits higher stability than the traditional decoction.

To achieve the object of the present disclosure, the present disclosure provides the following technical solutions: The present disclosure provides a method for preparing a Zhu Ling Tang extract solution, including the following steps:

soaking four traditional Chinese medicines in water to obtain a presoaked material solution, where the four traditional Chinese medicines include *Polyporus umbellatus, Poria cocos, Rhizoma Alismatis,* and *Talcum;* and during the soaking, a ratio of a mass of the water to a total mass of the four traditional Chinese medicines is less than or equal to 14:1;

conducting a first boiling extraction on the presoaked material solution to obtain a first aqueous extract solution and a medicinal residue;

mixing the medicinal residue with water, and conducting a second boiling extraction to obtain a second aqueous extract solution, where during the second boiling extraction, a ratio of a mass of the water to the total mass of the four traditional Chinese medicines is less than or equal to 12:1;

conducting a first concentration on the first aqueous extract solution to obtain a first concentrate; and combining the first concentrate with the second aqueous extract solution, and conducting a second concentration to obtain a second concentrate, where the first concentration and the second concentration each are conducted independently at a temperature of lower than or equal to 90°C; and

mixing the second concentrate and an aqueous solution of *Asini Corii Colla* to obtain the Zhu Ling Tang extract solution.

In some embodiments, during the soaking, the ratio of the mass of the water to the total mass of the four traditional Chinese medicines is in a range of (8-14):1; the soaking is conducted for 40 min to 60 min;

the first boiling extraction includes a heating stage under steam heating and a boiling holding stage under steam heating; the boiling holding stage is conducted with a heat preservation time of 40 min to 90 min, and the boiling holding stage is conducted under a steam pressure of lower than or equal to 0.1 MPa; and the heating stage is conducted under a steam pressure of lower than or equal to 0.2 MPa.

In some embodiments, during the second boiling extraction, the ratio of the mass of the water to the total mass of the four traditional Chinese medicines is in a range of (6-12): 1; and the second boiling extraction includes a heating stage under steam heating and a boiling holding stage under steam heating; the boiling holding stage is conducted with a heat preservation time of 25 min to 60 min, and the boiling holding stage is conducted under a steam pressure of lower than or equal to 0.1 MPa; and the heating stage is conducted under a steam pressure of lower than or equal to 0.2 MPa.

In some embodiments, a mass ratio of the *Polyporus umbellatus,* the *Poria cocos,* the *Rhizoma Alismatis,* and the *Talcum* is in a range of (0.7-1.3):(0.7-1.3):(0.7-1.3):(0.7-1.3).

In some embodiments, the first concentration and the second concentration each are conducted independently under steam heating;

the first concentration and the second concentration each are conducted at a temperature independently of 70°C to 90°C under a vacuum pressure independently of 0.03 MPa to 0.06 MPa; the first concentration and the second concentration each are conducted with a heat preservation time independently of less than or equal to 6 h; and the first concentration and the second concentration each are conducted under a steam pressure independently of 0.01 MPa to 0.05 MPa.

The present disclosure provides a method for preparing a Zhu Ling Tang extract paste, including the following step:

concentrating the Zhu Ling Tang extract solution prepared by the method according to the above technical solution to obtain the Zhu Ling Tang extract paste; where the concentrating is conducted at a temperature of lower than or equal to 90°C, and a relative density of the Zhu Ling Tang extract paste at 20°C is in a range of 1.12 to 1.16.

In some embodiments, the concentrating is conducted under steam heating;

the concentrating is conducted at a temperature of 70°C to 90°C under a vacuum pressure of 0.03 MPa to 0.06 MPa; the concentrating is conducted with a heat preservation time of less than or equal to 6 h; and the concentrating is conducted under a steam pressure of 0.01 MPa to 0.05 MPa.

The present disclosure provides a method for preparing a Zhu Ling Tang solid preparation, the Zhu Ling Tang solid preparation including a Zhu Ling Tang dry powder or a Zhu Ling Tang granule; where

under a condition that the Zhu Ling Tang solid preparation is the Zhu Ling Tang dry powder, the method includes: mixing the Zhu Ling Tang extract solution prepared by the method according to the above technical solution with an aqueous solution of an adjuvant, and concentrating a resulting mixture to obtain a mixed thick paste; and vacuum-drying the mixed thick paste to obtain the Zhu Ling Tang dry powder, where a mass ratio of the adjuvant to the Zhu Ling Tang extract solution is in a range of 1:50 to 7:50, and a mass of the Zhu Ling Tang extract solution is based on a total mass of the *Polyporus umbellatus,* the *Poria cocos,* the *Rhizoma Alismatis,* the *Talcum,* and the *Asini Corii Colla;* and under a condition that the Zhu Ling Tang solid preparation is the Zhu Ling Tang granule, the method includes: conducting dry granulation on the Zhu Ling Tang dry powder to obtain the Zhu Ling Tang granule.

In some embodiments, the vacuum-drying is conducted by vacuum belt drying, and working parameters for the vacuum belt drying include: a feeding temperature: 75°C to 85°C; a feeding rate: 2.0 L/h to 3.5 L/h; a running speed of a track: 150±50 mm/min; a rotational speed of a material-distributing motor: 100 r/min; an angle of a swingarm: 12° to 25°; heating temperatures: 95±5°C in a first zone, 96±5°C in a second zone, and 96±5°C in a third zone; a vacuum degree: 0.097 MP to 0.1 MP; a speed of a cutter: 10 s/time to 20 s/time; and a cooling temperature: 30±5°C; and

the dry granulation is conducted in a granulator, and working parameters for the dry granulation include: a pressure of a press roller of the granulator: 12 MPa to 20 MPa; a rotational speed of the press roller of the granulator: 4 r/min to 10 r/min; a vertical rotational speed of a screw feeder of the granulator: 25 r/min to 40 r/min; a horizontal rotational speed of the screw feeder of the granulator: 70 r/min to 120 r/min; and a mesh size of a sieve: 14 mesh.

**EP 4 595 947 A1**

The present disclosure provides a Zhu Ling Tang solid preparation prepared by the method according to the above technical solution, where a dry extract yield of the Zhu Ling Tang solid preparation is in a range of 18% to 23%; a total content of alisol B 23-acetate and alisol C 23-acetate in the Zhu Ling Tang solid preparation is in a range of 0.011 wt% to 0.020 wt%; and a total content of a donkey-derived polypeptide $A_1$ and a donkey-derived polypeptide $A_2$ in the Zhu Ling Tang solid preparation is in a range of 0.091 wt% to 0.18 wt%.

The present disclosure provides a method for preparing a Zhu Ling Tang extract solution, including the following steps: soaking four traditional Chinese medicines in water to obtain a presoaked material solution, where the four traditional Chinese medicines include *Polyporus umbellatus, Poria cocos, Rhizoma Alismatis,* and *Talcum,* and a ratio of a mass of the water to a total mass of the four traditional Chinese medicines is less than or equal to 14:1; conducting a first boiling extraction on the presoaked material solution to obtain a first aqueous extract solution and a medicinal residue; mixing the medicinal residue with water, and conducting a second boiling extraction to obtain a second aqueous extract solution, where a ratio of a mass of the water to the total mass of the four traditional Chinese medicines is less than or equal to 12:1; conducting a first concentration on the first aqueous extract solution to obtain a first concentrate; and combining the first concentrate with the second aqueous extract solution, and conducting a second concentration to obtain a second concentrate, where the first concentration and the second concentration each are conducted independently at a temperature of lower than or equal to 90°C; and mixing the second concentrate and an aqueous solution of *Asini Corii Colla* to obtain the Zhu Ling Tang extract solution. In the method for preparing the Zhu Ling Tang extract solution, the water extraction and concentration are comprehensively considered: the water extraction is reasonably designed to as a two-step process, and the amount of water used in each extraction step is set to no more than 14 times the weight of raw materials, so as to make the extraction rate of active pharmaceutical ingredients be improved by 20% to 30% compared to reference sample. Moreover, in the present disclosure, the extract solution is concentrated at a low temperature (lower than or equal to 90°C), which helps preserve the active pharmaceutical ingredients and avoids their destruction due to high temperatures. In summary, the Zhu Ling Tang extract solution prepared by the method of the present disclosure achieves consistent extraction efficiency with the traditional Zhu Ling Tang reference sample, and has high contents of effective active ingredients and excellent efficacy.

In some embodiments of the present disclosure, during the soaking, the ratio of the mass of the water to the total mass of the four traditional Chinese medicines is in a range of (8-14):1; the soaking is conducted for 40 min to 60 min; the first boiling extraction includes a heating stage under steam heating and a boiling holding stage under steam heating; the boiling holding stage is conducted with a heat preservation time of 40 min to 90 min, and the boiling holding stage is conducted under a steam pressure of lower than or equal to 0.1 MPa; and the heating stage is conducted under a steam pressure of lower than or equal to 0.2 MPa. In the conventional modern traditional Chinese medicine extraction, to pursue a high extraction rate, each extraction is often conducted for 1 h to 2 h. However, the extracted components are primarily impurities such as fibers and starches, with no significant increase in active ingredients. Moreover, the prolonged heating may even degrade the active ingredients. Compared with conventional modern extraction modes for traditional Chinese medicine, which often involve excessive extraction and high energy consumption, the method in the present disclosure achieves short extraction time and higher extraction efficiency, using a moderate amount of water in the first extraction process, thereby improving the quality and efficacy of a Zhu Ling Tang while reducing production costs.

In some embodiments of the present disclosure, during the second boiling extraction, the ratio of the mass of the water to the total mass of the four traditional Chinese medicines is in a range of (6-12):1; the second boiling extraction includes a heating stage under steam heating and a boiling holding stage under steam heating; the boiling holding stage is conducted with a heat preservation time of 25 min to 60 min, and the boiling holding stage is conducted under a steam pressure of lower than or equal to 0.1 MPa; and the heating stage is conducted under a steam pressure of lower than or equal to 0.2 MPa. The method achieves short extraction time and high extraction efficiency with moderate water in the second extraction process, enhancing the quality and efficacy of Zhu Ling Tang extract solution while reducing the production costs.

In some embodiments of the present disclosure, the first concentration and the second concentration each are conducted independently under steam heating at a temperature independently of 70°C to 90°C under a vacuum pressure independently of 0.03 MPa to 0.06 MPa; the first concentration and the second concentration each are conducted with a heat preservation time independently of less than or equal to 6 h; and the first concentration and the second concentration each are conducted under a steam pressure independently of 0.01 MPa to 0.05 MPa. The present disclosure adopts vacuum concentration with the temperature controlled at 70°C to 90°C, which improves evaporation efficiency and reduces degradation of active pharmaceutical ingredients compared to traditional atmospheric pressure concentration.

The present disclosure provides a method for preparing a Zhu Ling Tang extract paste, including the following steps: concentrating the Zhu Ling Tang extract solution prepared by the method according to the above technical solution to obtain the Zhu Ling Tang extract paste, where the concentrating is conducted at a temperature of lower than or equal to 90°C; and a relative density of the Zhu Ling Tang extract paste at 20°C is in a range of 1.12 to 1.16. The concentrating at a temperature of lower than or equal to 90°C in the present disclosure may reduce the destruction of active pharmaceutical ingredients.

5

The present disclosure provides a method for preparing a Zhu Ling Tang solid preparation, the Zhu Ling Tang solid preparation including a Zhu Ling Tang dry powder or a Zhu Ling Tang granule; where under a condition that the Zhu Ling Tang solid preparation is the Zhu Ling Tang dry powder, the method includes: mixing the Zhu Ling Tang extract solution prepared by the method according to the above technical solution with an aqueous solution of an adjuvant, and concentrating a resulting mixture to obtain a mixed thick paste; and vacuum-drying the mixed thick paste to obtain the Zhu Ling Tang dry powder, where a mass ratio of the adjuvant to the Zhu Ling Tang extract solution is in a range of (1-7):50, and a mass of the Zhu Ling Tang extract solution is based on a total mass of the *Polyporus umbellatus,* the *Poria cocos,* the *Rhizoma Alismatis,* the *Talcum,* and the *Asini Corii Colla;* and under a condition that the Zhu Ling Tang solid preparation is the Zhu Ling Tang granule, the method includes: conducting dry granulation on the Zhu Ling Tang dry powder to obtain the Zhu Ling Tang granule. The present disclosure provides an improved method for adjuvant addition in traditional granule production. Instead of adding adjuvants before granulation, the adjuvant is introduced as an aqueous solution prior to the concentration. This modification solves the problem of material loss during drying which typically occurs because the mixed thick paste (including *Asini Corii Colla,* polysaccharides, etc.) tends to adhere and cause blockages, and thus reduces a dry extract loss during the drying process. Unlike conventional methods where adjuvants are added before granulation, the method in the present disclosure ensures thorough mixing of the adjuvant with the Zhu Ling Tang extract solution. Traditional high-temperature drying and spray-drying often lead to agglomeration and may degrade active pharmaceutical ingredients. However, in the present disclosure, the vacuum-drying method adopted offers advantages such as low drying temperatures, short drying time, minimal loss, and high efficiency. During the drying process, the resulting Zhu Ling Tang solid preparation is not prone to agglomeration, and the active pharmaceutical ingredients would not be destructed. As a result, the components are evenly distributed in the Zhu Ling Tang solid preparation, and the Zhu Ling Tang solid preparation exhibits high efficacy.

The present disclosure provides a Zhu Ling Tang solid preparation prepared by the method according to the above technical solution, where a dry extract yield of the Zhu Ling Tang solid preparation is in a range of 18% to 23%; a total content of alisol B 23-acetate and alisol C 23-acetate in the Zhu Ling Tang solid preparation is in a range of 0.011 wt% to 0.020 wt%; and a total content of a donkey-derived polypeptide $A_1$ and a donkey-derived polypeptide $A_2$ in the Zhu Ling Tang solid preparation is in a range of 0.091 wt% to 0.18 wt%. In the present disclosure, the Zhu Ling Tang solid preparation demonstrates consistent quality standards with the Zhu Ling Tang reference sample, along with excellent solubility and high bioavailability. Compared to traditional decoctions, the Zhu Ling Tang solid preparation is more convenient to take and carry, and exhibits superior stability.

## BRIEF DESCRIPTION OF THE DRAWINGS

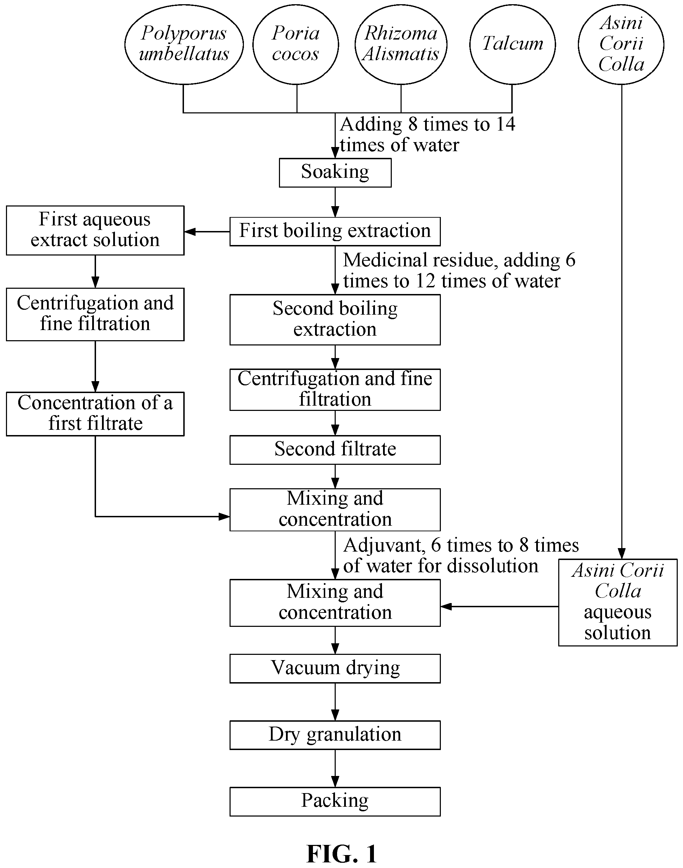

FIG. 1 shows a flowchart of the method for preparing a Zhu Ling Tang granule in an embodiment of the present disclosure; and

FIG. 2 shows high-performance liquid chromatography (HPLC) contrasted characteristic chromatograms for the Zhu Ling Tang granule in an embodiment of the present disclosure.

## DETAILED DESCRIPTION OF THE EMBODIMENTS

The present disclosure provides a method for preparing a Zhu Ling Tang extract solution, including the following steps:

soaking four traditional Chinese medicines in water to obtain a presoaked material solution, where the four traditional Chinese medicines include *Polyporus umbellatus, Poria cocos, Rhizoma Alismatis,* and *Talcum,* and during the soaking, a ratio of a mass of the water to a total mass of the four traditional Chinese medicines is less than or equal to 14:1;

conducting a first boiling extraction on the presoaked material solution to obtain a first aqueous extract solution and a medicinal residue;

mixing the medicinal residue with water, and conducting a second boiling extraction to obtain a second aqueous extract solution, where during the second boiling extraction, a ratio of a mass of the water to the total mass of the four traditional Chinese medicines is less than or equal to 12:1;

conducting a first concentration on the first aqueous extract solution to obtain a first concentrate, and combining the first concentrate with the second aqueous extract solution, and conducting a second concentration to obtain a second concentrate, where the first concentration and the second concentration each are conducted independently at a temperature of lower than or equal to 90°C; and

mixing the second concentrate and an aqueous solution of *Asini Corii Colla* to obtain the Zhu Ling Tang extract solution.

In the present disclosure, unless otherwise specified, all raw material components are commercially-available products well known to those skilled in the art.

In the present disclosure, four traditional Chinese medicines are soaked in water to obtain a presoaked material solution, where the four traditional Chinese medicines include *Polyporus umbellatus, Poria cocos, Rhizoma Alismatis,* and *Talcum,* and a ratio of a mass of the water to a total mass of the four traditional Chinese medicines is less than or equal to 14:1.

In some embodiments of the present disclosure, the *Polyporus umbellatus* is purchased from Shandong BaiWeiTang Chinese Herbal Medicine Drinks Slice Co., Ltd.

In some embodiments of the present disclosure, the *Poria cocos* is purchased from Anhui Xintai Pharmaceutical Co., Ltd.

In some embodiments of the present disclosure, the *Rhizoma Alismatis* is purchased from Shandong BaiWei-Tang Chinese Herbal Medicine Drinks Slice Co., Ltd.

In some embodiments of the present disclosure, the *Talcum* is purchased from Quzhou Nankong Chinese Traditional Medicine Co., Ltd.

In some embodiments of the present disclosure, the *Asini Corii Colla* is purchased from Dong-E-E-Jiao Co., Ltd.

In some embodiments of the present disclosure, a mass ratio of the *Polyporus umbellatus,* the *Poria cocos,* the *Rhizoma Alismatis,* and the *Talcum* is in a range of (0.7-1.3):(0.7-1.3):(0.7-1.3):(0.7-1.3), and more preferably is 1:1:1:1.

In some embodiments of the present disclosure, in parts by mass, a formula of the Zhu Ling Tang extract solution is as follows: 750 parts of *Polyporus umbellatus,* 750 parts of *Poria cocos,* 750 parts of *Rhizoma Alismatis,* 750 parts of *Talcum,* and 750 parts of *Asini Corii Colla.*

In some embodiments of the present disclosure, the *Polyporus umbellatus* is processed by a process as follows: (1) Selection for impurity removal. (2) Soaking at 5°C to 35°C for 0.5 h to 3 h. (3) Moistening at 5°C to 35°C for 3 h to 6 h. (4) Cutting to produce uniform thick slices of 2 mm to 4 mm. (5) Drying at 70°C to 80°C for 5 h to 6 h.

In some embodiments of the present disclosure, the *Poria cocos* is processed by a process as follows: (1) Selection for impurity removal.

In some embodiments of the present disclosure, the *Rhizoma Alismatis* is processed by a process as follows: (1) Selection for impurity removal. (2) Spray-washing for 10 min to 15 min. (3) Soaking at 15°C to 35°C for 5 h to 18 h. (4) Moistening at 15°C to 35°C for 18 h to 36 h. (5) Cutting to produce uniform thick slices of 2 mm to 4 mm. (6) Drying at 50°C to 60°C for 5 h to 6 h.

In some embodiments of the present disclosure, the *Talcum* is processed by a process as follows: (1) Selection for impurity removal. (2) Washing. (3) Drying at 70°C to 80°C for 3 h to 4 h. (4) Crushing with a crusher, and sieving through an 80-mesh medicinal sieve.

In some embodiments of the present disclosure, the *Talcum* requires wrapped decoction.

In some embodiments of the present disclosure, the soaking is conducted in an extraction tank.

In some embodiments of the present disclosure, during the soaking the ratio of the mass of the water to the total mass of the four traditional Chinese medicines is less than or equal to 14:1, preferably (8-12): 1, and more preferably (10-12): 1.

In some embodiments of the present disclosure, the soaking is conducted at room temperature.

In some embodiments of the present disclosure, the soaking is conducted for 40 min to 60 min and more preferably 50 min.

In the present disclosure, after the presoaked material solution is obtained, a first boiling extraction is conducted on the presoaked material solution to obtain a first aqueous extract solution and a medicinal residue.

In some embodiments of the present disclosure, the first boiling extraction is conducted by a process including: opening a steam valve of an extraction tank and heating with a heating steam; opening an external circulation of the extraction tank when a temperature reaches 90°C; starting timing when the temperature allows boiling; with a pressure of the heating steam stabilized, conducting the first boiling extraction; after the first boiling extraction is completed, closing the external circulation, and discharging a resulting medicinal solution to obtain the first aqueous extract solution and the medicinal residue.

In some embodiments of the present disclosure, the first boiling extraction includes a heating stage under steam heating and a boiling holding stage under steam heating.

In some embodiments of the present disclosure, when the presoaked material solution is heated by steam, and the heating stage is conducted under a steam pressure of lower than or equal to 0.2 MPa.

In some embodiments of the present disclosure, during the first boiling extraction, the boiling holding stage is conducted under a steam pressure of lower than or equal to 0.1 MPa and more preferably 0.05 MPa to 0.08 MPa.

In some embodiments of the present disclosure, during the first boiling extraction, the boiling holding stage is conducted with a heat preservation time of 40 min to 90 min and more preferably 40 min to 50 min.

In some embodiments of the present disclosure, after the first boiling extraction is completed, a post-treatment is conducted on a first medicinal solution obtained after the first boiling extraction to obtain the first aqueous extract solution. In some embodiments of the present disclosure, the post-treatment includes: filtering the first medicinal solution and then subjecting a resulting material to centrifugal separation to obtain a liquid component, and subjecting the liquid component to fine filtration to obtain the first aqueous extract solution. In some embodiments of the present disclosure, the filtration is conducted with a 120-mesh sieve, and the centrifugal separation is conducted in a centrifuge, where a separation speed for the centrifugal separation is 30 L/min to 40 L/min, and during the centrifugal separation, residue discharge is conducted once every 20 min. In some embodiments of the present disclosure, the fine filtration is conducted with a plate and frame filter, where a filter membrane of the plate and frame filter has a pore size of 5 μm to 15 μm.

In the present disclosure, after the medicinal residue is obtained, the medicinal residue is mixed with water, and a second boiling extraction is conducted to obtain a second aqueous extract solution,where during the second boiling extraction, a ratio of a mass of the water to the total mass of the four traditional Chinese medicines is less than or equal to 12:1.

In some embodiments of the present disclosure, during the second boiling extraction, the ratio of the mass of the water to the total mass of the four traditional Chinese medicines is (8-10):1.

In some embodiments of the present disclosure, the second boiling extraction is conducted by a process including: opening a steam valve of an extraction tank, and heating with a heating steam; opening an external circulation of the extraction tank when a temperature reaches 90°C;. starting timing when the temperature allows boiling; with a pressure of the heating steam stabilized, conducting the second boiling extraction; after the second boiling extraction is completed, closing the external circulation, and discharging a resulting medicinal solution to obtain the second aqueous extract solution.

In some embodiments of the present disclosure, the second boiling extraction includes a heating stage under steam heating and a boiling holding stage under steam heating.

In some embodiments of the present disclosure, when a medicinal residue mixture obtained by mixing the medicinal residue and the water is heated by heating steam, and the heating stage is conducted under a steam pressure of lower than or equal to 0.2 MPa.

In some embodiments of the present disclosure, during the second boiling extraction, the boiling holding stage is conducted under a steam pressure of lower than or equal to 0.1 MPa and more preferably 0.05 MPa to 0.08 MPa.

In some embodiments of the present disclosure, during the second boiling extraction, the boiling holding stage is conducted with a heat preservation time of 25 min to 60 min and more preferably 25 min to 40 min.

In some embodiments of the present disclosure, after the second boiling extraction is completed, a post-treatment is conducted on a second medicinal solution obtained after the second boiling extraction to obtain the second aqueous extract solution. In some embodiments of the present disclosure, the post-treatment includes: filtering the second medicinal solution,and then subjecting a resulting material to centrifugal separation to obtain a liquid component, and subjecting the liquid component to fine filtration to obtain the second aqueous extract solution. In some embodiments of the present disclosure, the filtration is conducted with a 120-mesh sieve, and the centrifugal separation is conducted in a centrifuge, where a separation speed for the centrifugal separation is 30 L/min to 40 L/min, and during the centrifugal separation, residue discharge is conducted once every 20 min. In some embodiments of the present disclosure, the fine filtration is conducted with a plate and frame filter, where a filter membrane of the plate and frame filter has a pore size of 5 μm to 15 μm.

In the present disclosure, after the first aqueous extract solution and the second aqueous extract solution are obtained, a first concentration is conducted on the first aqueous extract solution to obtain a first concentrate, and the first concentrate and the second aqueous extract solution are combined and subjected to a second concentration to obtain a second concentrate.

In some embodiments of the present disclosure, the first concentration is conducted under steam heating at a temperature of 70°C to 90°C, more preferably 75°C to 90°C, and most preferably 85°C under a vacuum pressure of 0.03 MPa to 0.06 MPa and more preferably 0.04 MPa to 0.05 MPa; the first concentration is conducted with a heat preservation time of less than or equal to 6 h; and the first concentration is conducted under a steam pressure of of 0.01 MPa to 0.05 MPa and more preferably 0.03 MPa to 0.045 MPa.

In some embodiments of the present disclosure, the second concentration is conducted under steam heating at a temperature of 70°C to 90°C, more preferably 75°C to 90°C, and most preferably 85°C under a vacuum pressure of 0.03 MPa to 0.06 MPa and more preferably 0.04 MPa to 0.05 MPa; the second concentration is conducted with a heat preservation time of less than or equal to 6 h; and the second concentration is conducted under a steam pressure of 0.01 MPa to 0.05 MPa and more preferably 0.03 MPa to 0.045 MPa.

In some embodiments of the present disclosure, a relative density of the second concentrate obtained after the second concentration at 20°C is 1.10 to 1.14. In the present disclosure, the relative density of the second concentrate refers to a density relative to water.

In the present disclosure, after the second concentrate is obtained, the second concentrate is mixed with an aqueous solution of *Asini Corii Colla* to obtain the Zhu Ling Tang extract solution.

In some embodiments of the present disclosure, in the aqueous solution of the *Asini Corii Colla,* a mass ratio of the *Asini Corii Colla* to water is in a range of 1:(2-6) and more preferably 1:(2.5-5).

In some embodiments of the present disclosure, the aqueous solution of the *Asini Corii Colla* is prepared by a process including the following steps: adding *Asini Corii Colla* to water and dissolving, and sieving to obtain an undersized part, which is the aqueous solution of the *Asini Corii Colla.* In some embodiments of the present disclosure, the dissolving is conducted under heating and stirring. The present disclosure has no special limitations on a speed and time of the stirring, as long as the *Asini Corii Colla* may be dissolved to produce a homogeneous *Asini Corii Colla* solution. In some embodiments of the present disclosure, the process further includes after the *Asini Corii Colla* is dissolved, removing foams and impurities in an upper layer of a resulting *Asini Corii* Colla-dissolved system, and then sieving. In some embodiments of the present disclosure, a sieve size for the sieving is 100 mesh.

The present disclosure has no special limitations on a specific implementation process for mixing the second concentrate with the aqueous solution of the *Asini Corii Colla,* as long as the raw materials may be mixed thoroughly.

The present disclosure provides a method for preparing a Zhu Ling Tang extract paste, including the following step:

concentrating (which is referred to as third concentrating hereinafter) the Zhu Ling Tang extract solution prepared by the method according to the above technical solution to obtain the Zhu Ling Tang extract paste; where the third concentrating is conducted at a temperature of lower than or equal to 90°C, and a relative density of the Zhu Ling Tang extract paste is in a range of 1.12 to 1.16 (20°C).

In some embodiments of the present disclosure, the third concentrating is conducted under steam heating at a temperature of 70°C to 90°C, more preferably 75°C to 90°C, and most preferably 85°C under a vacuum pressure of 0.03 MPa to 0.06 MPa and more preferably 0.04 MPa to 0.05 MPa; the third concentration is conducted with a heat preservation time of less than or equal to 6 h; and the third concentrating is conducted under a steam pressure of 0.01 MPa to 0.05 MPa and more preferably 0.03 MPa to 0.045 MPa.

In some embodiments of the present disclosure, a relative density of the Zhu Ling Tang extract paste is in a range of 1.12 to 1.16, where the relative density of the Zhu Ling Tang extract paste refers to a relative density at 20°C and a density relative to water.

The present disclosure provides a method for preparing a Zhu Ling Tang solid preparation, the Zhu Ling Tang solid preparation including a Zhu Ling Tang dry powder or a Zhu Ling Tang granule;where

under a condition that the Zhu Ling Tang solid preparation is the Zhu Ling Tang dry powder, the method includes: mixing the Zhu Ling Tang extract solution prepared by the method according to the above technical solution and an aqueous solution of an adjuvant,and concentrating a resulting mixture to obtain a mixed thick paste; where a mass ratio of the adjuvant to the Zhu Ling Tang extract solution is in a range of (1-7):50, and a mass of the Zhu Ling Tang extract solution is based on a total mass of the *Polyporus umbellatus,* the *Poria cocos,* the *Rhizoma Alismatis,* the *Talcum,* and the *Asini Corii Colla;* and vacuum-drying the mixed thick paste to obtain the Zhu Ling Tang dry powder; and

under a condition that the Zhu Ling Tang solid preparation is the Zhu Ling Tang granule, the method includes: conducting dry granulation on the Zhu Ling Tang dry powder to obtain the Zhu Ling Tang granule.

In the present disclosure, the Zhu Ling Tang dry powder is prepared by a process including the following steps: mixing the Zhu Ling Tang extract solution prepared by the method according to the above technical solution and an aqueous solution of an adjuvant, and concentrating (which is referred to as fourth concentrating hereinafter) a resulting mixture to obtain a mixed thick paste; where a mass ratio of the adjuvant to the Zhu Ling Tang extract solution is in a range of (1-7):50, and a mass of the Zhu Ling Tang extract solution is based on a total mass of the *Polyporus umbellatus,* the *Poria cocos,* the *Rhizoma Alismatis,* the *Talcum,* and the *Asini Corii Colla;* and vacuum-drying the mixed thick paste to obtain the Zhu Ling Tang dry powder.

In some embodiments of the present disclosure, the adjuvant is a pharmaceutically-acceptable adjuvant.

In some embodiments of the present disclosure, the adjuvant is at least one selected from the group consisting of dextrin, lactose, and soluble starch, and is more preferably lactose.

In some embodiments of the present disclosure, the mass ratio of the adjuvant to the Zhu Ling Tang extract solution is in a range of (1-7):50 and preferably (1.5-6.0):50.

In some embodiments of the present disclosure, the fourth concentrating is conducted under steam heating at a temperature of 70°C to 90°C and more preferably 75°C to 90°C under a vacuum pressure of 0.03 MPa to 0.06 MPa and more preferably 0.04 MPa to 0.05 MPa; the fourth concentrating is conducted with a heat preservation time of less than or equal to 6 h; and the fourth concentrating is conducted under a heating steam pressure of 0.01 MPa to 0.05 MPa and more preferably 0.03 MPa to 0.045 MPa.

In some embodiments of the present disclosure, a relative density of the mixed thick paste is in a range of 1.12 to 1.16, where the relative density of the mixed thick paste refers to a relative density at 20°C and a density relative to water.

In some embodiments of the present disclosure, the mixed thick paste obtained after the fourth concentrating is introduced into a transfer barrel and weighed for later use.

In some embodiments of the present disclosure, the vacuum-drying is conducted by vacuum belt drying, and working parameters for the vacuum belt drying include: a feeding temperature: 75°C to 85°C and more preferably 76°C to 82°C; a feeding rate: 2.0 L/h to 3.5 L/h and more preferably 2.2 L/h to 3.5 L/h; a running speed of a track: $150\pm50$ mm/min and more preferably $150\pm20$ mm/min; a rotational speed of a material-distributing motor: 100 r/min; an angle of a swingarm: 12° to 25° and more preferably 15° to 22°; heating temperatures: $95\pm5$°C in a first zone, $96\pm5$°C in a second zone, and $96\pm5$°C in a third zone; a vacuum degree: 0.097 MP to 0.1 MP and more preferably 0.098 MPa to 0.09 MPa; a speed of a cutter: 10 s/time to 20 s/time and more preferably 11 s/time to 18 s/time; and a cooling temperature: $30\pm5$°C.

In some embodiments of the present disclosure, a powder obtained after the vacuum-drying is collected by using a transfer barrel and packaged to obtain the Zhu Ling Tang dry powder for later use.

In some embodiments of the present disclosure, the Zhu Ling Tang granule is prepared by a process including the following step: conducting dry granulation on the Zhu Ling Tang dry powder to obtain the Zhu Ling Tang granule.

In some embodiments of the present disclosure, the dry granulation is conducted in a granulator, and working parameters for the dry granulation include: a pressure of a press roller of the granulator: 12 MPa to 20 MPa and more preferably 13 MPa to 16 MPa; a rotational speed of the press roller of the granulator: 4 r/min to 10 r/min and more preferably 5 r/min to 8 r/min; a vertical rotational speed of a screw feeder of the granulator:25 r/min to 40 r/min and more preferably 30 r/min to 38 r/min; a horizontal rotational speed of the screw feeder of the granulator: 70 r/min to 120 r/min and more preferably 80 r/min to 115 r/min; and a mesh size of a sieve: 14 mesh.

In some embodiments of the present disclosure, a granule obtained after the dry granulation is sieved and then placed in a transfer barrel for later use.

In some embodiments of the present disclosure, the granule is packaged by using a composite film at 6 g/bag, where the 6 g/bag means that an amount in each bag is controlled at 5.58 g to 6.42 g.

The present disclosure provides a Zhu Ling Tang solid preparation prepared by the method according to the above technical solution, where a dry extract yield of the Zhu Ling Tang solid preparation is in a range of 18% to 23%, a total content of alisol B 23-acetate and alisol C 23-acetate in the Zhu Ling Tang solid preparation is in a range of 0.011 wt% to 0.020 wt%, and a total content of a donkey-derived polypeptide $A_1$ and a donkey-derived polypeptide $A_2$ in the Zhu Ling Tang solid preparation is in a range of 0.091% to 0.18 wt%.

In some embodiments of the present disclosure, in parts by mass, raw materials for preparing 1,000 parts of the Zhu Ling Tang granule include: 750 parts of *Polyporus umbellatus,* 750 parts of *Poria cocos,* 750 parts of *Rhizoma Alismatis,* 750 parts of *Talcum,* 750 parts of *Asini Corii Colla,* and 100 parts to 400 parts of an adjuvant; where a mass of the adjuvant is in 120 parts to 380 parts and further preferably 140 parts to 360 parts.

In a specific embodiment of the present disclosure, the raw materials for preparing 1,000 g of the Zhu Ling Tang granule include: 750 g of the *Polyporus umbellatus,* 750 g of the *Poria cocos,* 750 g of the *Rhizoma Alismatis,* 750 g of the *Talcum,* 750 g of the *Asini Corii Colla,* and 100 g to 400 g of the adjuvant.

In some embodiments of the present disclosure, a mass of the adjuvant is 120 g to 380 g and further preferably 140 g to 360 g, and the adjuvant is at least one selected from the group consisting of dextrin, lactose, and soluble starch, and is more preferably lactose.

In some embodiments of the present disclosure, a specification of the Zhu Ling Tang granule is 6 g/bag.

In some embodiments of the present disclosure, the Zhu Ling Tang granule is packaged with a pharmaceutical composite film.

Some embodiments of present disclosure also provide a research and evaluation method for the preparation method of the Zhu Ling Tang solid preparation. The quality standard of the Zhu Ling Tang reference sample is taken as the benchmark, with the dry extract yield as the main evaluation index and the contents determined at different stages as auxiliary indexes. Specifically, contents of alisol B 23-acetate and alisol C 23-acetate are used as auxiliary indices during extraction, extraction solution post-treatment (purification), and concentration, while the contents of alisol B 23-acetate and alisol C 23-acetate, and characteristic polypeptides are used as auxiliary indexes during vacuum-drying and dry granulation. In some embodiments of the present disclosure, changes in the above-mentioned indices are monitored from raw materials feeding onward to systematically evaluate the preparation method of the Zhu Ling Tang solid preparation.

In some embodiments of the present disclosure, a detection method for the dry extract yield includes:

(1) Drying a measuring flask to constant weight

A clean measuring flask is taken, dried in an electric blast air oven at 105°C for 3 h with a cap opened, transferred to a dryer with the cap closed, cooled for 30 min, weighed accurately, further dried at the above temperature for 1 h, cooled for 30 min, and weighed. The difference between results of the two consecutive times of weighing does not exceed 0.3 mg.

(2) Determination

A determination method for an intermediate is as follows: An appropriate amount of a homogeneous test sample is taken, added to the measuring flask of constant weight, weighed accurately, steamed in a water bath until near dryness, transferred to a drying oven, dried at 105°C for 5 h, transferred to a dryer, cooled for 30 min, weighed accurately, dried for 1 h, transferred to a dryer, cooled for 30 min, and weighed once again. The difference between results of the two times of weighing does not exceed 5 mg.

A finished product is tested by using the oven-drying method (method II) under General Rule 0832 in the Chinese Pharmacopoeia, 2020 edition.

(3) Calculation

Intermediate:

$$\text{Dry extract yield (\%)} = \frac{(W2-W0) \times W3}{W1 \times W4} \times 100\%,$$

where W0 represents a constant weight of a measuring flask, g;
W1 represents a weight of a test sample, g;
W2 represents a constant weight of (measuring flask + test sample), g;
W3 represents a total weight of a sample, g; and
W4 represents a total weight of medicines, g.

Finished product

$$\text{Dry extract yield (\%)} = \frac{W3(1-\text{Sample moisture})}{W4} \times \frac{W6}{W6+W5(1-\text{Adjuvant moisture})} \times 100\%$$

where W3 represents a total weight of a sample, g;
W4 represents a total weight of medicines, g;
W5 represents a weight of an adjuvant, g; and
W6 represents a weight of a dry extract before the addition of an adjuvant, g.

In some embodiments of the present disclosure, contents of alisol B 23-acetate and alisol C 23-acetate is determined by using HPLC (General Rule 0512 in the Chinese Pharmacopoeia, 2020 edition).

Chromatographic conditions and system suitability testing: Octadecylsilane-bonded silica gel is adopted as a filler. Acetonitrile is adopted as a mobile phase A, and a 0.2% phosphoric acid solution is taken as a mobile phase B. Gradient elution (0 min to 15 min: 55%→70% A; 15 min to 30 min: 70%→80% A; 30 min to 35 min: 80%→95% A; and 35 min to 45 min: 95% A). Detection wavelengths are 208 nm and 246 nm. A column temperature is 30°C. A number of theoretical plates should not be less than 3,000 based on alisol B 23-acetate.

Preparation of a reference substance solution: Appropriate amounts of alisol B 23-acetate as a reference substance and alisol C 23-acetate as a reference substance are weighed and dissolved in methanol to prepare a mixed reference substance solution including 3 $\mu$g of alisol B 23-acetate and 2.5 $\mu$g of alisol C 23-acetate per 1 mL.

Preparation of a test sample solution: An appropriate amount of a sample is accurately weighed and added to a 50 mL measuring bottle, and an equal amount of acetonitrile is added to produce a mixture. The mixture is weighed, ultrasonically treated for 30 min, and cooled, and then 50% acetonitrile is added to a specified scale. The measuring bottle is fully shaken, and filtration is conducted. 25 mL of a subsequent filtrate is taken and subjected to evaporation to dryness to produce a residue. The residue is dissolved with 50% acetonitrile, diluted to 10 mL, and filtered, and a subsequent filtrate is collected.

Determination method: 10 $\mu$L of each of the reference substance solution and the test sample solution is accurately pipetted, injected into a liquid chromatograph, and tested.

In some embodiments of the present disclosure, a content of characteristic polypeptides is determined according to HPLC-mass spectrometry (General Rule 0512 and General Rule 0431 in the Chinese Pharmacopoeia, 2020 edition).

Chromatography and mass spectrometry conditions and system suitability testing: Octadecylsilane-bonded silica gel is adopted as a filler (an inner diameter of a chromatographic column is 2.1 mm). Acetonitrile is adopted as a mobile phase A and a 0.1% formic acid solution is adopted as a mobile phase B. Gradient elution (0 min to 15 min: 5%→ 14% A) is adopted. A flow rate is 0.3 mL/min.

Multiple reaction monitoring (MRM) is conducted with a triple quadrupole mass spectrometer in a positive ion mode of electrospray ionization (ESI). The monitored ion pairs are shown in Table 1.

Table 1 Monitored ion pairs of the triple quadrupole mass spectrometer

| Determined component | Quantitative ion pair, m/z | Qualitative ion pair, m/z |
|---|---|---|
| Donkey-derived polypeptide $A_1$ | 469.25 (double charge) → 712.30 | 469.25 (double charge) → 783.40 |
| Donkey-derived polypeptide $A_2$ | 618.25 (double charge) → 779.40 | 618.25 (double charge) → 850.40 |

A number of theoretical plates should not be less than 4,000 based on a peak of the donkey-derived polypeptide $A_1$.

Preparation of a reference substance solution: Appropriate amounts of a donkey-derived polypeptide $A_1$ as a reference substance and a donkey-derived polypeptide $A_2$ as a reference substance are weighed accurately, and a 1% ammonium bicarbonate solution is added to prepare a mixed solution at a concentration of 2.5 μg/1 mL.

Preparation of a test sample solution: An appropriate amount of a sample is weighed accurately and added to a 25 mL measuring flask, 20 mL of a 1% ammonium bicarbonate solution is added, and an ultrasonic treatment (with a power of 250 W and a frequency of 40 kHz) is conducted for 30 min. A1% ammonium bicarbonate solution is added to a specified scale, and the measuring flask is fully shaken to produce a solution. 200 μL of the solution is taken and added to a microcentrifuge tube, 200 μL of a 5 mg/mL trypsin solution and 600 μL of a 1% ammonium bicarbonate solution are added, and the microcentrifuge tube is fully shaken. Isothermal enzymatic hydrolysis is allowed at 37°C for 12 h. Filtration is conducted, and a subsequent filtrate is collected.

Determination method: 1 mL, 2 mL, 5 mL, 10 mL, 20 mL, and 25 mL of the reference substance solution are accurately taken and added to 50 mL measuring flasks, respectively, and then a 1% ammonium bicarbonate solution is added to a specified scale, so as to prepare standard curve solutions. 5 μL of each of the standard curve solutions with different concentrations and the test sample solution is accurately pipetted and injected into an HPLC-mass spectrometry instrument. A standard curve is plotted with a peak area of the reference substance as a y-coordinate and a concentration of the reference substance as an x-coordinate. A content of a donkey-derived polypeptide $A_1$ and a donkey-derived polypeptide $A_2$ in the test sample solution is calculated according to the standard curve.

Some embodiments of the present disclosure also provide quality control standards for the Zhu Ling Tang solid preparation described in the above technical solution, including an HPLC characteristic chromatogram, identification and content determination of characteristic polypeptides, and a dry extract yield for the Zhu Ling Tang solid preparation.

In some embodiments, the quality control standards for the Zhu Ling Tang solid preparation include characteristics, particle size, solubility, load difference, and microbial limit requirements for a granule.

In some embodiments of the present disclosure, in parts by mass, a formula of a Zhu Ling Tang granule is as follows: 750 parts of *Polyporus umbellatus,* 750 parts of *Poria cocos,* 750 parts of *Rhizoma Alismatis,* 750 parts of *Talcum,* and 750 parts of *Asini Corii Colla.*

In some embodiments of the present disclosure, a preparation method of the Zhu Ling Tang granule is performed with reference to the method for preparing the Zhu Ling Tang granule provided in the above technical solutions.

In some embodiments of the present disclosure, characteristics of the Zhu Ling Tang granule are as follows: a light-brown to dark-brown color, and a slightly-bitter and slightly-sweet taste.

In some embodiments of the present disclosure, an identification method for the Zhu Ling Tang granule is as follows: (1) 4 g of a Zhu Ling Tang granular powder is taken, 50 mL of diethyl ether is added, and an ultrasonic treatment is conducted for 10 min. Filtration is conducted to produce a filtrate, and the filtrate is subjected to evaporation to dryness to produce a residue. The residue is dissolved with 1 mL of methanol to produce a test sample solution. 1 g of each of medicines *Polyporus umbellatus* and *Poria cocos* as control medicines is taken to prepare control medicine solutions by the same method. According to the thin-layer chromatography (General Rule 0502 in the Chinese Pharmacopoeia, 2020 edition), 2 μL to 5 μL of each of the *Poria cocos* control medicine solution and the *Polyporus umbellatus* control medicine solution and 20 μL to 50 μL of the test sample solution A are pipetted and spotted on a same silica gel G thin-layer chromatography plate. With cyclohexane-ethyl acetate-formic acid (8:1:0.1) as a developing solvent, the developing is conducted. Then the silica gel G thin-layer chromatography plate is air-dried, and then subjected to fluorescence chromogenic development directly at 365 nm, or sprayed with 10% sulfuric acid in ethanol, air-dried, and then subjected to fluorescence chromogenic development at 365 nm. Spots in a same color appear at corresponding positions in the chromatography of a test sample and the chromatography of a control medicine.

(2) 2 g of a sample powder is taken, 50 mL of methanol is added, and an ultrasonic treatment is conducted for 10 min. Filtration is conducted to produce a filtrate, and the filtrate is subjected to evaporation to dryness to produce a residue.

The residue is dissolved with 1 mL of methanol to produce a test sample solution. 1 g of *Rhizoma Alismatis* as a control medicine is taken to prepare a control medicine solution by the same method. According to the thin-layer chromatography (General Rule 0502 in the Chinese Pharmacopoeia, 2020 edition), 2 μL of the control medicine solution and 5 μL of the test sample solution are pipetted and spotted on a same silica gel G thin-layer chromatography plate. With cyclohexane-ethyl acetate (1:1) as a developing solvent, the developing is conducted. The silica gel G thin-layer chromatography plate is air-dried, sprayed with a solution of 2% vanillin in sulfuric acid, and heated at 105°C until spots are clear. Spots with a same color occur at corresponding positions in a chromatography of a test sample and a chromatography of a control medicine.

(3) 0.1 g of a sample powder is weighed accurately and added to a 25 mL measuring flask, 20 mL of a 1% ammonium bicarbonate solution is added, and an ultrasonic treatment (with a power of 250 W and a frequency of 40 kHz) is conducted for 30 min. A 1% ammonium bicarbonate solution is added to a specified scale, and the measuring flask is fully shaken to produce a solution. 200 μL of the solution is taken and added to a microcentrifuge tube, 200 μL of a 1 mg/mL trypsin solution and 600 μL of a 1% ammonium bicarbonate solution are added, and the microcentrifuge tube is fully shaken. Isothermal enzymatic hydrolysis is allowed at 37°C for 12 h to produce a test sample solution. 0.1 g of *Asini Corii Colla* as a control medicine is taken to prepare a control medicine solution by the same method. A test is conducted according to the chromatography and mass spectrometry conditions for characteristic polypeptides under the content determination item, and mass-to-charge ratios (m/z) 539.8 (double charge) → 612.4 and (m/z) 539.8 (double charge) → 923.8 are selected as testing ion pairs. 5 μL of the *Asini Corii Colla* control medicine solution is taken and injected. Signal-to-noise ratios of MRM chromatographic peaks determined according to the above testing ion pairs both should be larger than 3:1.

5 μL of the test sample solution is pipetted and injected into an HPLC-mass spectrometry instrument, and tested. In the ion pair chromatography for the test sample determined with the mass-to-charge ratios (m/z) 539.8 (double charge) → 612.4 and (m/z) 539.8 (double charge) → 923.8 as testing ion pairs, a chromatographic peak with a consistent retention time with the control medicine should be presented.

In the present disclosure, the detection for the Zhu Ling Tang granule should conform to the relevant provisions for granules (General Rule 0104 in the Chinese Pharmacopoeia, 2020 edition).

In some embodiments of the present disclosure, a dry extract yield of the Zhu Ling Tang granule is determined according to the moisture determination method (General Rule 0832 in the Chinese Pharmacopoeia, 2020 edition), and the dry extract yield should be 18.0% to 23.0%.

In some embodiments of the present disclosure, a method for determining extracts in the Zhu Ling Tang granule is as follows: The Zhu Ling Tang granule is taken and finely ground to produce a ground material. About 2 g of the ground material is accurately weighed, and 100 mL of ethanol is accurately added. A content of extracts in the Zhu Ling Tang granule is determined by the hot extraction method under the alcohol-soluble extract determination (General Rule 2201 in the Chinese Pharmacopoeia, 2020 edition). The content of extracts in the Zhu Ling Tang granule should be 12.0% to 23.5%.

In some embodiments of the present disclosure, a characteristic chromatogram of the Zhu Ling Tang granule is determined by HPLC (General Rule 0512 in the Chinese Pharmacopoeia, 2020 edition).

Chromatography conditions and system suitability testing: Octadecylsilane-bonded silica gel is adopted as a filler. Acetonitrile is adopted as a mobile phase A and a 0.2% phosphoric acid solution is adopted as a mobile phase B. Gradient elution is conducted as specified in Table 2. A detection wavelength is 208 nm. A column temperature is 30°C. A number of theoretical plates should not be less than 2,000 based on a peak of alisol B.

Table 2 Gradient elution program

| Time (min) | Mobile phase A (%) | Mobile phase B (%) |
|---|---|---|
| 0-15 | 55→70 | 45→30 |
| 15-30 | 70→85 | 30→15 |
| 30-35 | 85→95 | 15→5 |
| 35-45 | 95 | 5 |

Preparation of a reference substance solution: Alisol A, alisol B, and alisol B 23-acetate are accurately weighed and dissolved with acetonitrile to prepare a mixed reference substance solution with an alisol A mass concentration of 40 μg/mL, an alisol B mass concentration of 25 μg/mL, and an alisol B 23-acetate mass concentration of 23 μg/mL.

Preparation of a test sample solution: 1.0 g of a sample powder is accurately weighed and added to an Erlenmeyer flask with a stopper, 50 mL of acetonitrile is accurately added, and the Erlenmeyer flask is stoppered. An ultrasonic treatment (with a power of 800 W and a frequency of 40 kHz) is conducted for 30 min, cooling is allowed, and filtration is conducted to produce a filtrate. The filtrate is subjected to evaporation to dryness to produce a residue. The residue is dissolved with 80% acetonitrile, diluted to 5 mL, and filtered to produce the test sample solution.

Determination method: 20 μL of each of the reference substance solution and the test sample solution is accurately pipetted, injected into a liquid chromatograph, and tested.

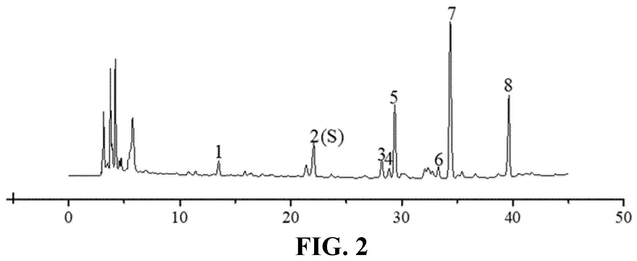

There should be 8 characteristic peaks in a characteristic chromatogram of the test sample. 3 peaks should correspond to the retention times of peaks for the corresponding reference substances. A peak corresponding to a peak of the alisol B reference substance should be an S peak. A relative retention time of each characteristic peak relative to the S peak is calculated. The relative retention time should be within ±10% of a specified value. Specified values are as follows: 0.61 (peak 1), 1.00 (peak 2), 1.28 (peak 3), 1.31 (peak 4), 1.34 (peak 5), 1.52 (peak 6), 1.57 (peak 7), and 1.81 (peak 8). Contrasted characteristic chromatograms are shown in FIG. 2. In FIG. 2, a peak 1 is for alisol A, a peak S is for alisol B, and a peak 3 is for alisol B 23-acetate.

In some embodiments of the present disclosure, an amino acid content in the Zhu Ling Tang granule is determined by HPLC (General Rule 0512 in the Chinese Pharmacopoeia, 2020 edition).

Chromatography conditions and system suitability testing: Octadecylsilane-bonded silica gel is adopted as a filler. An acetonitrile-0.1 mol/L sodium acetate solution (a pH is adjusted with acetic acid to 6.5) (7:93) is adopted as a mobile phase A, and acetonitrile-water (4:1) is adopted as a mobile phase B. Gradient elution is conducted as specified in Table 3. A flow rate is 0.8 mL/min. A detection wavelength is 254 nm. A column temperature is 43°C. A number of theoretical plates should not be less than 5,000 based on a peak of L-hydroxyproline.

Table 3 Gradient elution program for amino acid content determination

| Time (min) | Mobile phase A (%) | Mobile phase B (%) |
|---|---|---|
| 0-11 | 100→93 | 0→7 |
| 11-13.9 | 93→88 | 7→12 |
| 13.9-14 | 88→85 | 12→15 |
| 14-29 | 85→66 | 15→34 |
| 29-30 | 66→0 | 34→100 |

Preparation of a reference substance solution: Appropriate amounts of L-hydroxyproline, glycine, alanine, and proline are weighed accurately, and a 0.1 mol/L hydrochloric acid solution is added to prepare a mixed solution including 80 μg of L-hydroxyproline, 0.16 mg of glycine, 70 μg of alanine, and 0.12 mg of proline per 1 mL.

Preparation of a test sample solution: 0.2 g of a sample powder is weighed accurately and added to a 25 mL measuring flask, 20 mL of a 0.1 mol/L hydrochloric acid solution is added, and an ultrasonic treatment (with a power of 500 W and a frequency of 40 kHz) is conducted for 30 min. Cooling is allowed, then a 0.1 mol/L hydrochloric acid solution is added to a specified scale, and the measuring flask is fully shaken to produce a solution. 2 mL of the solution is accurately taken and added to a 10 mL ampoule, then 2 mL of hydrochloric acid is added, and hydrolysis is allowed at 150°C for 1 h. A resulting hydrolysis system is cooled, transferred to an evaporation dish, and washed with 10 mL of water in batches. Resulting washing solutions are combined in an evaporation dish, and subjected to evaporation to dryness to produce a residue. The residue is dissolved with a 0.1 mol/L hydrochloric acid solution, transferred to a 25 mL measuring flask, diluted with a 0.1 mol/L hydrochloric acid solution to a specified scale, and thoroughly shaken.

The reference substance solution and the test sample solution each are accurately taken in 5 mL and added to a 25 mL measuring flask, 2.5 mL of a solution of 0.1 mol/L phenyl isothiocyanate (PITC) in acetonitrile and 2.5 mL of a solution of 1 mol/L triethylamine in acetonitrile are added, and the measuring flask is thoroughly shaken and placed at room temperature for 1 h. 50% acetonitrile is added to a specified scale, and the measuring flask is thoroughly shaken to produce a mixed solution. 10 mL of the mixed solution is taken, 10 mL of n-hexane is added, and a resulting mixture is shaken and placed for 10 min. A liquid in a lower layer is collected and filtered, and a subsequent filtrate is collected.

Determination method: 5 μL of each of the derived reference substance and test sample solutions is accurately pipetted, injected into a liquid chromatograph, and tested.

In some embodiments of the present disclosure, each bag should include 0.25 g to 0.55 g of *Asini Corii Colla* based on L-hydroxyproline, 0.50 g to 1.05 g of *Asini Corii Colla* based on glycine, 0.20 g to 0.45 g of *Asini Corii Colla* based on alanine, and 0.30 g to 0.60 g of *Asini Corii Colla* based on proline.

In some embodiments of the present disclosure, a content of characteristic polypeptides in the Zhu Ling Tang granule is determined by HPLC-mass spectrometry (General Rule 0512 and General Rule 0431 in the Chinese Pharmacopoeia, 2020 edition).

Chromatography and mass spectrometry conditions and system suitability testing: Octadecylsilane-bonded silica gel is adopted as a filler (an inner diameter of a chromatographic column is 2.1 mm). Acetonitrile is adopted as a mobile phase A and a 0.1% formic acid solution is adopted as a mobile phase B. Gradient elution is conducted as specified in Table 4. A flow rate is 0.3 mL/min.

Table 4 Elution program for characteristic polypeptide determination

| Time (min) | Mobile phase A (%) | Mobile phase B (%) |
|---|---|---|
| 0-15 | 5→14 | 95→86 |

MRM is conducted with a triple quadrupole mass spectrometer in a positive ion mode of ESI. The monitored ion pairs are shown in Table 5.

Table 5 Monitored ion pairs of triple quadrupole mass spectrometry for characteristic polypeptides

| Determined component | Quantitative ion pair, m/z | Qualitative ion pair, m/z |
|---|---|---|
| Donkey-derived polypeptide $A_1$ | 469.25 (double charge) → 712.30 | 469.25 (double charge) → 783.40 |
| Donkey-derived polypeptide $A_2$ | 618.25 (double charge) → 779.40 | 618.25 (double charge) → 850.40 |

A number of theoretical plates should not be less than 4,000 based on a peak of the donkey-derived polypeptide $A_1$.

Preparation of a reference substance solution: Appropriate amounts of a donkey-derived polypeptide $A_1$ as a reference substance and a donkey-derived polypeptide $A_2$ as a reference substance are weighed accurately, and a 1% ammonium bicarbonate solution is added to prepare a mixed solution at a concentration of 2.5 $\mu$g/1 mL.

Preparation of a test sample solution: 0.1 g of a sample powder is weighed accurately and added to a 25 mL measuring flask, 20 mL of a 1% ammonium bicarbonate solution is added, and an ultrasonic treatment (with a power of 250 W and a frequency of 40 kHz) is conducted for 30 min. A 1% ammonium bicarbonate solution is added to a specified scale, and the measuring flask is fully shaken to produce a solution. 200 $\mu$L of the solution is taken and added to a microcentrifuge tube, 200 $\mu$L of a 1 mg/mL trypsin solution and 600 $\mu$L of a 1% ammonium bicarbonate solution are added, and the microcentrifuge tube is fully shaken. Isothermal enzymatic hydrolysis is allowed at 37°C for 12 h. Filtration is conducted, and a subsequent filtrate is collected.

Determination method: 1 mL, 2 mL, 5 mL, 10 mL, 20 mL, and 25 mL of the reference substance solution are accurately taken and added to 50 mL measuring flasks, respectively, and then a 1% ammonium bicarbonate solution is added to a specified scale, so as to prepare standard curve solutions. 5 $\mu$L of each of the standard curve solutions with different concentrations and the test sample solution is accurately pipetted and injected into an HPLC-mass spectrometry instrument. A standard curve is plotted with a peak area of the reference substance as a y-coordinate and a concentration of the reference substance as an x-coordinate. A content of a donkey-derived polypeptide $A_1$ and a donkey-derived polypeptide $A_2$ in the test sample solution is calculated according to the standard curve.

In some embodiments of the present disclosure, each bag should include 5.5 mg to 11 mg of *Asini Corii Colla* based on a total amount of the donkey-derived polypeptide $A_1$ and the donkey-derived polypeptide $A_2$.

In some embodiments of the present disclosure, the functions and indications of the Zhu Ling Tang granule are as follows: diuresis, yin nourishment, and heat clearing. The Zhu Ling Tang granule may be used for dysuria, fever, thirst with a desire to drink, or restlessness and insomnia, or accompanying cough, nausea/vomiting, diarrhea, or red tongue with white or slightly-yellow coating, and thready and rapid pulse, and may also be used for strangury with hematuria, painful and hesitant urination, dribbling urination, and lower abdominal distension/pain.

In some embodiments of the present disclosure, the usage and dosage of the Zhu Ling Tang granule is as follows: The Zhu Ling Tang granule is dissolved in boiling water and then taken orally 3 times daily with one bag each time.

In some embodiments of the present disclosure, a specification of the Zhu Ling Tang granule is 6 g/bag.

In some embodiments of the present disclosure, the Zhu Ling Tang granule is stored in a sealed state.

The technical solutions of the present disclosure will be clearly and completely described below with reference to the examples of the present disclosure. Apparently, the described examples are merely some rather than all of the examples of the present disclosure. All other examples obtained by those of ordinary skill in the art based on the examples of the present disclosure without inventive efforts shall fall within the scope of the present disclosure.

In the examples and comparative examples of the present disclosure:

Sources of Chinese herbal slices: *Polyporus umbellatus, Poria cocos, Talcum,* and *Asini Corii Colla* are consistent with the sources in the Chinese Pharmacopoeia, 2020 edition. *Rhizoma Alismatis* is a dried rhizome of *Alisma orientale* (Sam.) Juzep of *Alisma* L. of *Alismataceae.*

Each traditional Chinese medicine is processed before use:

A processing method for the *Polyporus umbellatus* is as follows: (1) Selection for impurity removal. (2) Soaking at 5°C to 35°C for 0.5 h to 3 h. (3) Moistening at 5°C to 35°C for 3 h to 6 h. (4) Cutting to produce uniform thick slices of 2 mm to 4 mm.

(5) Drying at 70°C to 80°C for 5 h to 6 h.

A processing method for the *Poria cocos* is as follows: (1) Selection for impurity removal.

A processing method for the *Rhizoma Alismatis* is as follows: (1) Selection for impurity removal. (2) Spray-washing for 10 min to 15 min. (3) Soaking at 15°C to 35°C for 5 h to 18 h. (4) Moistening at 15°C to 35°C for 18 h to 36 h. (5) Cutting to produce uniform thick slices of 2 mm to 4 mm. (6) Drying at 50°C to 60°C for 5 h to 6 h.

A processing method for the *Talcum* is as follows: (1) Selection for impurity removal. (2) Washing. (3) Drying at 70°C to 80°C for 3 h to 4 h. (4) Crushing with a crusher, and sieving through an 80-mesh medicinal sieve.

**Example 1**

Raw materials for a Zhu Ling Tang granule prepared in this example of the present disclosure were as follows: 750 g of *Polyporus umbellatus,* 750 g of *Poria cocos,* 750 g of *Rhizoma Alismatis,* 750 g of *Talcum,* 750 g of *Asini Corii Colla,* and 100 g to 400 g of an adjuvant.

Sources of the raw materials:

*Polyporus umbellatus:* purchased from Shandong BaiWeiTang Chinese Herbal Medicine Drinks Slice Co., Ltd, China.

*Poria cocos:* purchased from Anhui Xintai Pharmaceutical Co., Ltd, China.

*Rhizoma Alismatis:* purchased from Shandong BaiWeiTang Chinese Herbal Medicine Drinks Slice Co., Ltd, China.

*Talcum:* purchased from Quzhou Nankong Chinese Traditional Medicine Co., Ltd, China.

*Asini Corii Colla:* purchased from Dong-E-E-Jiao Co., Ltd, China.

Adjuvant: purchased from Zhenjiang Kangfu Bioengineering Co., Ltd, China.

7.5 kg of each of *Polyporus umbellatus, Poria cocos, Rhizoma Alismatis,* and *Talcum* (wrapped decoction) was weighed and added to an extraction tank, water was added at an amount 8 times to 14 times an amount of the Chinese herbal slices, and soaking was conducted for 50 min to obtain a presoaked material solution. A steam valve of the extraction tank was opened, and heating was conducted with a heating steam at a pressure of lower than or equal to 0.20 MPa. When a temperature reached 90°C, an external circulation was opened. When the temperature allowed boiling, the timing was started. A steam pressure was stabilized at 0.05 MPa to 0.08 MPa for 40 min to 50 min, and then the external circulation was closed (first decoction) to obtain a first medicinal solution. The first medicinal solution was discharged, and a medicinal residue was obtained. The first medicinal solution was filtered through a 120-mesh filter and then pumped by using a pump into a centrifuge for separation. During the separation, a separation speed was controlled at 30 L/min to 40 L/min, and a resulting residue was discharged about once every 20 min. A centrifugate obtained each time was filtered through a plate and frame filter (a filter membrane had a pore size of 5 μm to 15 μm) to obtain a first aqueous extract solution.

Water was added at an amount 6 times to 12 times an amount of the medicines to the medicinal residue, and heating was conducted with a heating steam at a pressure of lower than or equal to 0.20 MPa. When a temperature reached 90°C, an external circulation was opened. When the temperature allowed boiling, the timing was started. A steam pressure was stabilized at 0.05 MPa to 0.08 MPa for 25 min to 40 min, then the external circulation was closed (second decoction) to obtain a second medicinal solution, and the second medicinal solution was discharged. The second medicinal solution was filtered through a 120-mesh filter and then pumped by using a pump into a centrifuge, and separation was conducted. During the separation, a separation speed was controlled at 30 L/min to 40 L/min, and a resulting residue was discharged about once every 20 min. A centrifugate obtained each time was filtered through a plate and frame filter (a filter membrane had a pore size of 5 μm to 15 μm) to obtain a second aqueous extract solution.

The second aqueous extract solution was introduced into a vacuum concentrator. A steam valve was opened, and heating was started. Concentration was conducted with a temperature of 70°C to 90°C, a vacuum pressure of 0.03 MPa to 0.06 MPa, a steam pressure of 0.03 MPa to 0.05 MPa, and a heat preservation time of less than or equal to 6 h to obtain a first concentrate. The first concentrate and the first aqueous extract solution were combined and introduced into a vacuum concentrator. A steam valve was opened, and heating was started. Concentration was conducted with a temperature of 70°C to 90°C, a vacuum pressure of 0.03 MPa to 0.06 MPa, a steam pressure of 0.01 MPa to 0.05 MPa, and a heat preservation time of less than or equal to 6 h to obtain a second concentrate for later use. A relative density of the second concentrate was 1.10 to 1.14 (20°C). 7.5 kg of *Asini Corii Colla* was weighed, and water was added in a mass 2 times to 6 times a mass of the *Asini Corii Colla.* Heating was conducted until boiling, and stirring was conducted constantly, such that the *Asini Corii Colla* was dissolved to obtain a homogeneous *Asini Corii Colla* solution. The homogeneous *Asini Corii Colla* solution was filtered through a 100-mesh sieve to obtain an *Asini Corii Colla* aqueous solution for later use. 1.0 kg to 4.0 kg of an adjuvant was weighed and poured into a stainless-steel barrel, water was added in a mass 2 times to 6 times a mass of the adjuvant, and stirring was conducted for complete dissolution to obtain an adjuvant aqueous solution for later use.

The second concentrate, the *Asini Corii Colla* aqueous solution, and the adjuvant aqueous solution were introduced into a single-effect concentrator. A steam valve was opened, and heating was started. Concentration was conducted with a temperature of 70°C to 90°C, a vacuum pressure of 0.03 MPa to 0.06 MPa, a steam pressure of 0.01 MPa to 0.05 MPa, and a heat preservation time of less than or equal to 6 h to obtain a mixed thick paste with a relative density of 1.12 to 1.16 (20°C). The mixed thick paste was introduced into a transfer barrel and weighed for later use.

The mixed thick paste was vacuum-dried in a vacuum belt dryer to obtain a Zhu Ling Tang dry powder. Working parameters for the vacuum belt dryer were as follows: a feeding temperature: 75°C to 85°C; a feeding speed: 2.0 L/h to 3.5 L/h; a running speed of a track: 150±50 mm/min; an angle of a swingarm: 12° to 25°; a speed of a material-distributing motor: 100 r/min; heating temperatures: 95±5°C in a first zone, 96±5°C a second zone, and 96±5°C in a third zone; a cooling temperature: 30±5°C; a speed of a cutter: 10 s/time to 20 s/time; and a vacuum degree: 0.097 MPa to 0.1 MPa. The Zhu Ling Tang dry powder was collected in a transfer barrel and packaged for later use.

The Zhu Ling Tang dry powder was taken, weighed, transferred to a granulator, and granulated to obtain a Zhu Ling Tang granule for later use. Parameters for the granulator were as follows: a pressure of a press roller: 12 MPa to 20 MPa; rotational speeds of a screw feeder: a vertical rotational speed: 25 r/min to 40 r/min, and a horizontal rotational speed: 70 r/min to 120 r/min; a rotational speed of the press roller: 4 r/min to 10 r/min; and a mesh size of a sieve: 14 mesh. The Zhu Ling Tang granule was sieved and placed in a transfer barrel for later use. The Zhu Ling Tang granule was packed with a composite film at 6 g/bag. The 6 g/bag indicated that an amount in each bag was controlled at 5.58 g to 6.42 g.

**Example 2**

Research on a preparation process of a Zhu Ling Tang granule

1. Experimental object

With a quality standard of a Zhu Ling Tang reference sample as a benchmark and a dry extract yield and contents of alisol B 23-acetate and alisol C 23-acetate as evaluation indexes, the number of extractions, the extraction time, and the water amount for a Zhu Ling Tang preparation were investigated and determined.

2. Experimental protocol

Chinese herbal slices for Zhu Ling Tang (55.2 g of each of *Polyporus umbellatus, Poria cocos, Rhizoma Alismatis,* and *Talcum* except for *Asini Corii Colla)* were taken and added to a round-bottomed flask, an appropriate amount of water was added, and soaking was conducted for 50 min. The round-bottomed flask was placed in an intelligent thermostatic electric heating mantle, and reflux extraction was conducted to obtain an extraction solution. A dry extract yield and contents of alisol B 23-acetate and alisol C 23-acetate of a resulting decoction were detected to determine process parameters. The investigated extraction parameters are shown in Table 6.

Through a single-factor experiment, the number of extractions, the extraction time, and the water amount were investigated and determined successively.

Table 6 Parameters investigated for an extraction process of the Zhu Ling Tang granule

| Investigation item | Number of extractions | Extraction time | Water amount |
|---|---|---|---|
| Investigation content | 1 | 45 min, 30 min, 30 min | 6 times and 4 times |
| | 2 | 60 min, 40 min, 30 min | 8 times and 6 times |
| | 3 | 90 min, 60 min, 30 min | 10 times and 8 times |
| | - | - | 12 times and 10 times |
| | - | - | 14 times and 12 times |

3. Experimental method

3.1 Experimental method to investigate the number of extractions: Chinese herbal slices for Zhu Ling Tang (55.2 g of each of *Polyporus umbellatus, Poria cocos, Rhizoma Alismatis,* and *Talcum* except for *Asini Corii Colla)* were taken and added to a round-bottomed flask, an appropriate amount of water was added, and soaking was conducted for 50 min. The round-bottomed flask was placed in an intelligent thermostatic electric heating mantle, and reflux extraction was conducted three times. A first extraction was conducted for 45 min with water at an amount 14 times an amount of the Chinese herbal slices. A second extraction and a third extraction each were conducted for 30 min with water at an amount 12 times the amount of the Chinese herbal slices. For an extraction solution obtained each time, a dry extract yield and index components were detected.

3.2 Experimental method to investigate the extraction time: Chinese herbal slices for Zhu Ling Tang (55.2 g of each of *Polyporus umbellatus, Poria cocos, Rhizoma Alismatis,* and *Talcum* except for *Asini Corii Colla*) were taken and added to a round-bottomed flask, an appropriate amount of water was added, and soaking was conducted for 50 min. The round-bottomed flask was placed in an intelligent thermostatic electric heating mantle, and reflux extraction was conducted three times. The extraction time are shown in Table 6. A first extraction was conducted with water at an amount 14 times an amount of the Chinese herbal slices. A second extraction and third extraction each were conducted with water at an amount 12 times the amount of the Chinese herbal slices.

3.3 Experimental method to investigate the water amount: Chinese herbal slices for Zhu Ling Tang (55.2 g of each of *Polyporus umbellatus, Poria cocos, Rhizoma Alismatis,* and *Talcum* except for *Asini Corii Colla*) were taken and added to a round-bottomed flask, an appropriate amount of water was added, and soaking was conducted for 50 min. The round-bottomed flask was placed in an intelligent thermostatic electric heating mantle, and reflux extraction was conducted twice. The extraction time was determined according to "3.2", and the water amount was referred to Table 6.

4. Experimental results

4.1 Investigation results for the number of extractions are shown in Table 7.

Table 7 Investigation results for the number of extractions for the Zhu Ling Tang granule (n = 2)

| Batch No. | Procedure | Dry extract yield (%) | Index component content (%) | | |
| --- | --- | --- | --- | --- | --- |
| | | | Alisol B 23-acetate | Alisol C 23-acetate | Alisol B 23-acetate + Alisol C 23-acetate |
| ZLKEXS2202001 | First extraction | 3.65 | 0.0141 | 0.0078 | 0.0219 |
| | Second extraction | 1.95 | 0.0067 | 0.0033 | 0.0100 |
| | Third extraction | 1.32 | 0.0064 | 0.0019 | 0.0083 |
| Reference sample | | 4.60 | 0.0139 | 0.0062 | 0.0201 |
| Note: The content (%) refers to a converted content in the *Rhizoma Alismatis* medicines. | | | | | |

Results in Table 7: (1) Dry extract yield: A dry extract yield (3.65%) of the first extraction for the Zhu Ling Tang granule is lower than a requirement of the reference sample (dry extract yield: 4.60%). A total dry extract yield of the first extraction and the second extraction (5.60%) is 21.7% higher than the requirement of the reference sample. A total dry extract yield of the first extraction, the second extraction, and the third extraction (6.80%) is 50% higher than the requirement of the reference sample. From the perspective of compensating for the loss in the later procedures (about 20% to 30%) and the energy loss during production in a modern workshop, two extractions are selected.

(2) Contents of alisol B 23-acetate and alisol C 23-acetate resulting from the first extraction both are higher than the corresponding contents of the reference sample. With the current water amount, the extraction of index components is relatively complete, and the number of extractions does not affect the index components.

4.2 Investigation results for the extraction time are shown in Table 8.

Table 8 Investigation results for the extraction time (n = 2)

| Batch No. | Process | Experimental conditions | Dry extract yield (%) | Index component content (%) | | |
| --- | --- | --- | --- | --- | --- | --- |
| | | | | Alisol B 23-acetate | Alisol C 23-acetate | Alisol B 23-acetate + Alisol C 23-acetate |
| ZLKLXS2202002 | First extraction | 45 min, 30 min, 30 min | 4.40 | 0.0155 | 0.0083 | 0.0238 |
| | Second extraction | | 2.00 | 0.0084 | 0.0034 | 0.0118 |
| | Third extraction | | 1.33 | 0.0046 | 0.0014 | 0.0060 |
| ZLKLXS2202003 | First extraction | 60 min, 40 min 30 min | 4.19 | 0.0095 | 0.0059 | 0.0154 |
| | Second extraction | | 2.13 | 0.0077 | 0.0030 | 0.0107 |
| | Third extraction | | 1.14 | 0.0033 | 0.0012 | 0.0045 |

(continued)

| Batch No. | Process | Experimental conditions | Dry extract yield (%) | Index component content (%) | | |
| --- | --- | --- | --- | --- | --- | --- |
| | | | | Alisol B 23-acetate | Alisol C 23-acetate | Alisol B 23-acetate + Alisol C 23-acetate |
| ZLKLXS2202004 | First extraction | 90 **min,** 60 min, 30 min | 4.97 | 0.0131 | 0.009 | 0.0221 |
| | Second extraction | | 1.96 | 0.0072 | 0.0031 | 0.0103 |
| | Third extraction | | 0.99 | 0.0045 | 0.0012 | 0.0057 |
| Reference sample | - | 45 min | 4.60 | 0.0139 | 0.0062 | 0.0201 |
| Note: The content (%) refers to a converted content in the *Rhizoma Alismatis* medicines. | | | | | | |

Results in Table 8: When the extraction time is extended, the dry extract yield and the content indexes do not increase significantly. Under each of the three different extraction times, a total dry extract yield of the first extraction and the second extraction exceeds a total dry extract yield of the three extractions by 80%. Two extractions are determined, and the corresponding extraction times are 45 min and 30 min, respectively.

4.3 Investigation results for the water amount are shown in Table 9.

Table 9 Investigation results for the water amount

| Batch No. | Sample | Water amount (times) | Dry extract yield (%) | Index component content (%) | | |
| --- | --- | --- | --- | --- | --- | --- |
| | | | | Alisol B 23-acetate | Alisol C 23-acetate | Alisol B 23-acetate + Alisol C 23-acetate |
| ZLKLXS2203006 | First extraction | 6 | 3.47 | 0.0077 | 0.0065 | 0.0142 |
| | Second extraction | 4 | 1.64 | 0.0065 | 0.0048 | 0.0113 |
| ZLKLXS2203007 | First extraction | 8 | 3.77 | 0.0085 | 0.0076 | 0.0161 |
| | Second extraction | 6 | 1.72 | 0.0067 | 0.0049 | 0.0116 |
| ZLKLXS2203008 | First extraction | 10 | 4.01 | 0.0117 | 0.0075 | 0.0192 |
| | Second extraction | 8 | 1.65 | 0.0085 | 0.0045 | 0.0130 |
| ZLKLXS2203009 | First extraction | 12 | 4.42 | 0.0132 | 0.0099 | 0.0231 |
| | Second extraction | 10 | 1.50 | 0.0074 | 0.0042 | 0.0116 |
| ZLKLXS2203010 | First extraction | 14 | 4.16 | 0.0170 | 0.0088 | 0.0258 |
| | Second extraction | 12 | 1.71 | 0.0096 | 0.0036 | 0.0132 |
| Averages for a reference sample | | 14.5 | 4.60 | 0.0132 | 0.0062 | 0.0201 |
| Note: The content (%) refers to a converted content in the *Rhizoma Alismatis* medicines. | | | | | | |

Results in Table 9: (1) Dry extract yield: With the increase of the water amount, the dry extract yield gradually increases. Total dry extract yields of the first extraction and the second extraction for batches 006 to 010 are 11.1%, 19.3%, 23.0%, 28.7%, and 27.6% higher than the corresponding total dry extract yield of the reference sample, respectively. There is no significant difference in the dry extract yield between the water amounts of 8 times to 14 times during the first extraction (RSD%: less than 5). From the perspective of making up for the loss in the later procedures (about 20% to 30%), the water amount of 10 times to 14 times is determined.

(2) Content indexes: As the water amount of extraction increases, the index contents gradually increase. At different water amounts, total content indexes of the first extraction and the second extraction are higher than the corresponding total content index of the reference sample. When the water amount is of 10 times and 12 times, a content of alisol B 23-acetate does not change basically, and exceeds the corresponding content of the reference sample by 50%. Contents of alisol C 23-acetate at the different water amounts are not different from each other significantly, and all are 50% higher than the corresponding content of the reference sample, which can make up for the loss in the subsequent procedures. To comprehensively consider the extraction efficiency and make up for the loss in the subsequent procedures, the water amount of 10 times to 14 times is selected.

5. Experimental conclusion

In summary, parameters for the extraction process of the Zhu Ling Tang granule are as follows: two extractions are adopted, including a first extraction and a second extraction. In the first extraction, a water amount of 10 times to 14 times is adopted, and the decoction is conducted for 40 min to 90 min. In the second extraction, a water amount of 8 times to 12 times is adopted, and the decoction is conducted for 25 min to 60 min.

**Example 3**

Investigation of a concentration mode

1. Experimental object

The impacts of a concentration temperature and a concentration time on a dry extract yield, index component contents, and a characteristic chromatogram for the Zhu Ling Tang granule were investigated to determine the parameter ranges for a concentration process of the Zhu Ling Tang granule.

2. Experimental method

2.1 Investigation of a concentration temperature

1 L of an extraction solution for the Zhu Ling Tang granule was taken, weighed, and then vacuum-concentrated in a rotary evaporator. The changes in a dry extract yield and index component contents after the concentration for 2 h at 70°C, 80°C, 90°C, and 95°C were investigated.

2.2 Investigation of a concentration time

1 L of an extraction solution for the Zhu Ling Tang granule was taken, weighed, and then vacuum-concentrated in a rotary evaporator. The changes in a dry extract yield and index component contents after the concentration for 2 h, 4 h, and 6 h at 90°C were investigated.

3. Experimental results

3.1 Investigation results for the concentration temperature are shown in Table 10.

Table 10 Investigation results for the concentration temperature

| Sample | Dry extract yield | | Alisol B 23-acetate | | Alisol C 23-acetate | | Alisol B 23-acetate + Alisol C 23-acetate | |
|---|---|---|---|---|---|---|---|---|
| | (%) | RSD (%) | Content (%) | **RSD** (%) | Content (%) | RSD (%) | Content (%) | **RSD** (%) |
| Original medicinal solution | 4.18 | | 0.0094 | | 0.0087 | | 0.0181 | |
| 70°C | 4.22 | | 0.0081 | | 0.0086 | | 0.0167 | |
| 80°C | 4.1 | 1.46 | 0.0093 | 7.04 | 0.0089 | 1.87 | 0.0182 | 3.89 |
| 90°C | 4.09 | | 0.0082 | | 0.0087 | | 0.0169 | |
| 95°C | 4.09 | | 0.0085 | | 0.009 | | 0.0175 | |
| Note: The content (%) refers to a converted content in the *Rhizoma Alismatis* medicines. | | | | | | | | |

Results in Table 10: (1) Dry extract yield: At the different concentration temperatures, the dry extract yield of the Zhu Ling Tang granule does not change significantly (RSD%: 1.46).
(2) Index components: The different concentration temperatures have a great impact on a content of alisol B 23-acetate in the Zhu Ling Tang granule (RSD%: 7.04), no significant impact on a content of alisol C 23-acetate (RSD%: 1.87), and no significant impact on a total content of alisol B 23-acetate and alisol C 23-acetate (RSD%: 3.89), indicating that alisol B 23-acetate in the Zhu Ling Tang granule is sensitive to a temperature.

The experimental results indicate that a concentration temperature of 70°C to 95°C does not significantly affect the dry extract yield and index components of the Zhu Ling Tang granule. Therefore, a concentration temperature range is determined to be 70°C to 95°C.

3.2 Investigation results for a concentration time are shown in Table 11.

Table 11 Investigation results for the concentration time

| Sample | Dry extract yield | | Alisol B 23-acetate | | Alisol C 23-acetate | | Alisol B 23-acetate + Alisol C 23-acetate | |
|---|---|---|---|---|---|---|---|---|
| | (%) | RSD (%) | Content (%) | RSD (%) | Content (%) | RSD (%) | Content (%) | **RSD** (%) |
| Original medicinal solution | 6.35 | 4.39 | 0.0279 | 3.18 | 0.0039 | 6.77 | 0.0314 | 3.50 |
| Concentration for 2 h | 5.92 | | 0.0268 | | 0.0034 | | 0.0302 | |
| Concentration for 4 h | 5.98 | | 0.0261 | | 0.0030 | | 0.0291 | |
| Concentration for 6 h | 6.47 | | 0.0261 | | 0.0032 | | 0.0293 | |

Results in Table 11: (1) Dry extract yield: Under the different concentration times, the dry extract yield of the Zhu Ling Tang granule does not change significantly (RSD%: 4.39).

Index components: The concentration times of 2 h, 4 h, and 6 h have no significant impact on a content of alisol B 23-acetate in the Zhu Ling Tang granule (RSD%: 3.18), a significant impact on a content of alisol C 23-acetate (RSD%: 6.77), and no significant impact on a total content of alisol B 23-acetate and alisol C 23-acetate (RSD%: 3.50), indicating that alisol C 23-acetate is sensitive to a concentration time.

The experimental results show that the concentration at 90°C for 6 h has no influence on the dry extract yield and index components of the Zhu Ling Tang granule. It indicates that the extraction solution for the Zhu Ling Tang granule can be vacuum-concentrated for no more than 6 h.

4. Experimental conclusion

After the influence of different concentration temperatures and different concentration times on the Zhu Ling Tang granule is investigated with the dry extract yield and the contents of alisol B 23-acetate and alisol C 23-acetate as evaluation indexes, parameters of the concentration process for the Zhu Ling Tang granule are determined as follows: a concentration temperature range: 70°C to 95°C, and a concentration time: no more than 6 h.

**Example 4**

100 L extraction tank experiment

In order to further verify the applicability of the parameters for the extraction and concentration processes to modern production equipment, three batches of small-scale testing verification were carried out with a 100 L extraction tank.

**1. Experimental process**

1.1 Soaking and extraction

1.4490 kg of *Polyporus umbellatus,* 1.4490 kg of *Poria cocos,* 1.4490 kg of *Rhizoma Alismatis,* 1.4490 kg of *Talcum,* and 1.4490 kg of *Asini Corii Colla* were weighed. The *Talcum* was filled in a decoction bag, and the decoction bag was tied at a suitable position. The medicines other than *Asini Corii Colla* were placed in the 100 L extraction tank, water was added at an amount 10 times an amount of the Chinese herbal slices, and soaking was conducted for 50 min. A first extraction under heat was conducted for 45 min, and a resulting medicinal solution was discharged. Water was added at an amount 8 times the amount of the Chinese herbal slices. A second extraction under heat was conducted for 30 min, and a resulting medicinal solution was discharged.

1.2 Post-treatment (purification) of extraction solutions

The above extraction solutions were combined, centrifuged by using a centrifuge, and filtered by using a plate and frame filter.

1.3 Concentration

A medicinal solution obtained after fine filtration was fed into a 100 L single-effect concentrator, and concentrated with a steam pressure of 0.01 MPa to 0.03 MPa, a vacuum pressure of 0.02 MPa to 0.04 MPa, and a temperature of 80°C to 90°C. During the concentration, the condition of the medicinal solution needed to be observed constantly. The concentration was conducted until a relative density was 1.004 to 1.007 (20°C). The *Asini Corii Colla* was dissolved and introduced into the single-effect concentrator, and the concentration was continued until a relative density was 1.12 to 1.18 (20°C).

2. Experimental results

According to the three consecutive batches of extraction testing in the 100 L extraction tank for the Zhu Ling Tang granule, the main devices for extraction, purification, concentration, etc. exhibited excellent compatibility, the key process parameters were in line with the controlled ranges, and the entire testing process was smooth. Three batches of concentrates produced for the Zhu Ling Tang granule all have a dry extract yield in line with the standard of the Zhu Ling Tang reference sample and have higher index component contents than the reference sample, with small differences (for the dry extract yields and the contents of alisol B 23-acetate and alisol C 23-acetate of the three batches of finished products, RSD% is less than 5). Results are shown in Table 12.

Table 12 Results of the three batches of 100 L-scale extraction testing

| Batch No. | Dry extract yield | | Alisol B 23-acetate | | Alisol C 23-acetate | | Alisol B 23-acetate + Alisol C 23-acetate | |
|---|---|---|---|---|---|---|---|---|
| | (%) | RSD (%) | Content (%) | RSD (%) | Content (%) | RSD (%) | Content (%) | RSD (%) |
| ZLKLXS2203011 | 21.34 | | 0.0153 | | 0.0129 | | 0.0282 | |
| ZLKLXS2203012 | 20.98 | 1.97 | 0.0145 | 3.27 | 0.0113 | 7.33 | 0.0258 | 4.46 |
| ZLKLXS2203013 | 21.82 | | 0.0200 | | 0.0115 | | 0.0269 | |
| Reference sample | 18.0-23.0 | | 0.0092-0.0172 | | 0.0043-0.0081 | | 0.014-0.026 | |
| Note: The content (%) refers to a converted content in the *Rhizoma Alismatis* medicines. | | | | | | | | |

**Example 5**

Pilot-scale production testing

The production process route and major parameters for the Zhu Ling Tang granular preparation were basically determined through the small-scale optimization. In order to further verify the rationality of the production facilities and the process route, three batches of pilot-scale testing verification were carried out with a 500 L extraction tank, which was specifically as follows:

**1. Testing process**

1.1 Soaking and extraction

7.452 kg of *Polyporus umbellatus,* 7.452 kg of *Poria cocos,* 7.452 kg of *Rhizoma Alismatis,* and 7.452 kg of *Talcum* were weighed. The *Talcum* was filled in a decoction bag, and the decoction bag was tied at a suitable position. The medicines were placed in a 500 L extraction tank. Water was added at an amount 10 times an amount of the Chinese herbal slices, and soaking was conducted for 50 min. A first extraction under heat was conducted for 45 min, and a resulting medicinal solution was discharged. Water was added at an amount 8 times the amount of the Chinese herbal slices. A second extraction under heat was conducted for 30 min, and a resulting medicinal solution was discharged.

1.2 Post-treatment (purification) of extraction solutions

The medicinal solutions were combined, centrifuged by using a centrifuge, and subjected to fine filtration by using a plate and frame filter.

1.3 Concentration

A medicinal solution obtained after the fine filtration was fed into a vacuum concentrator and concentrated with a temperature of 70°C to 90°C, a vacuum degree of 0.03 MPa to 0.06 MPa, and a steam pressure of lower than or equal to 0.09 MPa. During the concentration, the boiling and temperature were observed at any time, and the vacuum pressure and steam pressure were adjusted. The concentration was conducted until a concentrate with a relative density of 1.004 to 1.007 (20°C) was obtained for later use.

1.4 Dissolution *of Asini Corii Colla* and an adjuvant

7.452 kg of *Asini Corii Colla* was weighed and placed in a jacketed pot, and water was added at an amount about 2 times to 6 times an amount of the *Asini Corii Colla.* Heating was conducted until boiling, and stirring was conducted continuously to obtain a homogeneous *Asini Corii Colla* solution. Foams and impurities in an upper layer were removed, and a resulting solution was filtered through a 100-mesh sieve to obtain an *Asini Corii Colla* solution for later use.

3.000 kg of the adjuvant (40% of a dry extract) was weighed and poured into a stainless-steel barrel, water was added at an amount about 2 times to 6 times an amount of the adjuvant, and stirring was conducted for complete dissolution to produce an obtain solution for later use.

1.5 Mixing and concentration

The concentrate, the *Asini Corii Colla* solution, and the adjuvant solution were introduced into a single-effect concentrator. Concentration was conducted to obtain a thick paste with a relative density of 1.12 to 1.16 (20°C). The thick paste was introduced into a transfer barrel and weighed for later use.

1.6 Drying

The thick paste was introduced into a storage tank, heated to 75°C to 85°C, and dried in a vacuum belt dryer to obtain a powder. Parameters of the vacuum belt dryer were as follows: a feeding temperature: 75°C to 85°C; a feeding speed: 2.0 L/h to 3.5 L/h; a running speed of a track: $150\pm50$ mm/min; an angle of a swingarm: 12° to 25°; a speed of a material-distributing motor: 100 r/min; heating temperatures: $95\pm5$°C in a first zone, $96\pm5$°C in a second zone, $96\pm5$°C in a third zone, and $30\pm5$°C (cooling), in a fourth zone; a speed of a cutter: 10 s/time to 20 s/time; and a vacuum degree: 0.097 MPa to 0.1 MPa. The powder was collected in a transfer barrel and packaged for later use.

1.7 Granulation

The powder obtained after the belt drying was taken, weighed, transferred to a granulator, and granulated to obtain a granule for later use. Parameters for the granulator were as follows: a pressure of a press roller: 12 MPa to 20 MPa; rotational speeds of a screw feeder: a vertical rotational speed: 20 r/min to 40 r/min, and a horizontal rotational speed: 70 r/min to 120 r/min; a rotational speed of the press roller: 4 r/min to 10 r/min; and a mesh size of a sieve: 14 mesh. During the granulation, the conditions of the granulator and the characteristics of the granule were observed, the granulation parameters were adjusted appropriately, and the powder was fed and the granule was collected in time. A power supply for a vibrating sieve was turned on. the The granule obtained after the granulation was placed in a hopper, and a valve of the hopper was adjusted, such that a feeding speed allowed the complete separation of qualified particles from a fine powder. The qualified particles were placed in a transfer barrel for later use. The fine powder was returned for the granulation.

2. Experimental results

According to the three consecutive batches of pilot-scale production testing for the Zhu Ling Tang preparation, the process route was reasonable, the major devices for extraction, centrifugation, fine filtration, concentration, drying, granulation, etc. exhibited excellent compatibility, the key process parameters were in line with the controlled ranges, and the entire testing process was smooth. Three batches of Zhu Ling Tang preparations produced have a dry extract yield and index component contents in line with the standards of the Zhu Ling Tang reference sample, with small differences (for the dry extract yields, contents of alisol B 23-acetate and alisol C 23-acetate, and characteristic polypeptides (donkey-derived

polypeptides $A_1$ and $A_2$) of the three batches of finished products, RSD% is less than 5). Results are shown in Table 13.

Table 13 Experimental results of the three batches of commercial-scale production testing

| Batch No. | Dry extract yield | | Alisol B 23-acetate + Alisol C 23-acetate | | Donkey-derived polypeptides $A_1$ +$A_2$ | |
|---|---|---|---|---|---|---|
| | (%) | RSD (%) | Content (%) | **RSD** (%) | Content (%) | **RSD** (%) |
| ZLKLZS2205014 | 21.96 | | 0.0303 | | 0.1504 | |
| ZLKLZS2205015 | 21.02 | 2.74 | 0.0297 | 3.11 | 0.1668 | 4.23 |
| ZLKLZS2205016 | 20.89 | | 0.0285 | | 0.1598 | |
| Reference sample | 18.0-23.0 | | 0.014-0.026 | | 0.11-0.20 | |

Notes: The content (%) of Alisol B 23-acetate + Alisol C 23-acetate refers to a converted content in the *Rhizoma Alismatis* medicine. The content of donkey-derived polypeptides $A_1$ + $A_2$ refers to a converted content in the *Asini Corii Colla* medicine.

**Comparative Example 1**

100 L extraction tank (reference sample method) test

Based on the requirement that the Zhu Ling Tang preparation should have basically the same quality standards as the reference sample, in order to ensure the consistent quality between the preparation and the reference sample, the preparation method for the reference sample was directly used to produce the preparation.

The preparation method for the reference sample was as follows: 13.8 g of each of *Polyporus umbellatus, Poria cocos, Rhizoma Alismatis,* and *Talcum* was taken, 800 mL of water was added, decoction was conducted, and filtration was conducted to obtain about 400 mL of a decoction solution. 13.8 g of *Asini Corii Colla* was added and dissolved by gentle heating. Vacuum concentration was conducted to about 200 mL, and lyophilization was conducted to obtain a Zhu Ling Tang reference sample.

The preparation method for the reference sample had the following characteristics: no soaking, water amount of 14.5 times, and a single time of extraction.

A preparation was produced in a 100 L extraction tank directly with the preparation method for the reference sample. The applicability of the method was evaluated with a dry extract yield and contents of alisol B 23-acetate and alisol C 23-acetate of an extraction solution as indexes. An experimental process was as follows:

1. Experimental process

1.1 Extraction

1.035 kg of *Polyporus umbellatus,* 1.035 kg of *Poria cocos,* 1.035 kg of *Rhizoma Alismatis,* and 1.035 kg of *Talcum* were weighed. The *Talcum* was filled in a decoction bag, and the decoction bag was tied at a suitable position. Chinese herbal slices were placed in a 100 L extraction tank. Water was added at an amount 14.5 times an amount of the Chinese herbal slices. Extraction was conducted for 45 min, and a resulting medicinal solution was discharged.

1.2 Purification

A resulting extraction solution was centrifuged by using a centrifuge and then filtered by using a plate and frame filter.

1.3 Concentration

A medicinal solution obtained after fine filtration was fed into a 100 L single-effect concentrator, and subjected to concentration with a steam pressure of 0.01 MPa to 0.03 MPa, a vacuum pressure of 0.02 MPa to 0.04 MPa, and a temperature of 80°C to 90°C. During the concentration, the condition of the medicinal solution needed to be observed constantly. The concentration was conducted until a relative density was 1.004 (20°C). The *Asini Corii Colla* was dissolved and introduced into the single-effect concentrator, and the concentration was continued until a relative density was 1.042 (20°C).

2. Test results

A concentrate for the Zhu Ling Tang granule produced directly by the reference sample method in the 100L extraction tank has a dry extract yield within the range for the reference sample, but has lower index component contents than the reference sample. The dry extract yield is 7.8% lower than dry extract yields of the determined Zhu Ling Tang granule processes (batch No.: ZLKLXS2203011-ZLKLXS2203013). Results are shown in Table 14. Table 14 shows that the reference sample method in the modern technology has a low extraction rate, and could not meet the quality standard requirements of the reference sample. Thus, it is necessary to study and determine a process suitable for modem production equipment, such that the prepared Zhu Ling Tang granule meets the standards of the Zhu Ling Tang reference sample.

Table 14 Results of the 100 L extraction tank (reference sample method) test

| Batch No. | Dry extract yield | | Alisol B 23-acetate | | Alisol C 23-acetate | | Alisol B 23-acetate + Alisol C 23-acetate | |
|---|---|---|---|---|---|---|---|---|
| | (%) | RSD (%) | Content (%) | RSD (%) | Content (%) | RSD (%) | Content (%) | **RSD** (%) |
| ZLKLXS2203011 | 21.34 | | 0.0153 | | 0.0129 | | 0.0282 | |
| ZLKLXS2203012 | 20.98 | 1.97 | 0.0145 | 3.27 | 0.0113 | 7.33 | 0.0258 | 4.46 |
| ZLKLXS2203013 | 21.82 | | 0.0200 | | 0.0115 | | 0.0269 | |
| ZLKLXS2202005 | 19.71 | | 0.004 | | 0.0083 | | 0.0123 | |
| Reference sample | 18.8-23.0 | | 0.0092-0.0172 | | 0.0043-0.0081 | | 0.014-0.026 | |
| Note: The content (%) refers to a converted content in the *Rhizoma Alismatis* medicine. | | | | | | | | |

In the present disclosure, the method for preparing the modem Zhu Ling Tang granule preparation based on the classic formula Zhu Ling Tang includes processing Chinese herbal slices, soaking, extraction, and post-treatment (purification), concentration, drying, granulation of the extraction solution, etc. The method ensures a stable and controllable production process, and produces granules with consistent quality standard compared to the Zhu Ling Tang reference sample prepared using the ancient method. Moreover, the Zhu Ling Tang preparation has advantages such as high stability, portability, and convenient taking over traditional decoction.

In the present disclosure, the processing method and quality standard for Chinese herbal slices for preparing the Zhu Ling Tang solid preparation ensure stable and uniform quality of the medicines, thereby controlling the quality of the preparation from the source.

In conventional extraction methods for Chinese patent drugs, excessive extraction and high energy consumption are common issues due to the pursuit of maximum extraction rate Each extraction is conducted for 1 h to 2 h, but the extracted components are often impurities such as fibers and starches, and the yield of active ingredients does not increase. Moreover, prolonged heating may even degrade some ingredients. In contrast, in the present disclosure, the method accounts for losses during purification, concentration, and drying to prepare granules that meet the quality standards of the reference sample. The soaking time, the water amount, the number of extractions, and the extraction time are optimized to achieve a 20% to 30% higher extraction rate compared to the reference sample. As a result, the extraction method ensures comparable quality to traditional decoction while reducing production costs.

In the present disclosure, the entire process after the extraction is performed at low temperatures (lower than 100°C). During purification, physical methods such as centrifugation and plate and frame filtration are used for impurity removal. Vacuum concentration is adopted for concentration, and vacuum belt drying for drying. Low temperature is conducive to the protection and retention of components, and could avoid the destruction of components due to high temperatures. Thus, the potential damage during industrial production is minimized, and the consistency between traditional decoction and modern preparation is ensured.

In the present disclosure, a concentration process for a Zhu Ling Tang extract paste is conducted at a temperature of 70°C to 90°C, with a concentration time not exceeding 6 h.

In the present disclosure, the traditional method of adding an adjuvan in granule preparation (where the adjuvant is added before granulation and then thorough mixing is conducted) is modified by introducing the adjuvant during the concentration. The addition of an adjuvant during the concentration solves the problem that there is prone to material loss during drying because medicinal solution for the Zhu Ling Tang preparation (including *Asini Corii Colla,* polysaccharides, etc.) is adhesive and is easy to cause blockage. Compared with the conventional addition of an adjuvant before granulation, the addition of an adjuvant during the concentration could allow the thorough mixing of an adjuvant with raw materials. Moreover, an extraction yield of the Zhu Ling Tang granule is low, and the addition of an adjuvant at the concentration stage could relatively reduce the dry extract loss during belt drying.

In the present disclosure, the drying for the Zhu Ling Tang solid preparation is conducted using vacuum belt drying, which offers advantages such as low drying temperature, short drying time, minimal loss, and high efficiency, making it particularly suitable for Zhu Ling Tang preparation materials with high viscosity and easy agglomeration.

In the present disclosure, a method for esearching and evaluating the preparation of the Zhu Ling Tang solid preparation adopts dry extract yield as the major evaluation index, with contents determined in stages as auxiliary indexes. The contents of alisol B 23-acetate and alisol C 23-acetate serve as auxiliary indexes during extraction, purification, and concentration, and the contents of alisol B 23-acetate and alisol C 23-acetate and the content of characteristic polypeptides are taken as auxiliary indexes at the belt drying and granulation stages. Changes in the indexes are tracked from raw material feeding to systematically evaluate each procedure.

In the present disclosure, the quality standards specify the detection methods for characteristics, identification, extracts, a characteristic chromatogram, and content determination of the Zhu Ling Tang preparation, along with the upper and lower limits for content determination.

In the present disclosure, a preparation quality control mode using a reference sample as a benchmark adopts a combination of dry extract yield, characteristic chromatogram, and index component content to comprehensively monitor the overall chemical composition of a standard decoction. The established multi-component control index is used for investigating the stability of products to ensure the commercialization of finished drugs.

In the present disclosure, a method is proposed to study the quantity-to-quality transfer rule based on raw materials → process → intermediate → preparation. The upper and lower limits for the contents of L-hydroxyproline, glycine, alanine, proline, a donkey-derived polypeptide $A_1$, and a donkey-derived polypeptide $A_2$ in Chinese herbal slices are formulated to ensure the quality of the preparation. The present disclosure is committed to building a quality standard system for controlling the whole process of medicines → Chinese herbal slices → intermediate → solid preparation.

The above are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

**Claims**

1. A method for preparing a Zhu Ling Tang extract solution, comprising:

   soaking four traditional Chinese medicines in water to obtain a presoaked material solution, wherein the four traditional Chinese medicines comprise *Polyporus umbellatus, Poria cocos, Rhizoma Alismatis,* and *Talcum;* and during the soaking, a ratio of a mass of the water to a total mass of the four traditional Chinese medicines is less than or equal to 14:1;

   conducting a first boiling extraction on the presoaked material solution to obtain a first aqueous extract solution and a medicinal residue;

   mixing the medicinal residue with water, and conducting a second boiling extraction to obtain a second aqueous extract solution, wherein during the second boiling extraction, a ratio of a mass of the water to the total mass of the four traditional Chinese medicines is less than or equal to 12:1;

   conducting a first concentration on the first aqueous extract solution to obtain a first concentrate; and combining the first concentrate with the second aqueous extract solution, and conducting a second concentration to obtain a second concentrate, wherein the first concentration and the second concentration each are conducted independently at a temperature of lower than or equal to 90°C; and

   mixing the second concentrate and an aqueous solution of *Asini Corii Colla* to obtain the Zhu Ling Tang extract solution.

2. The method for preparing the Zhu Ling Tang extract solution of claim 1, wherein during the soaking, the ratio of the mass of the water to the total mass of the four traditional Chinese medicines is in a range of 8:1 to 14:1; and the soaking is conducted for 40 minutes to 60 minutes; and

   the first boiling extraction comprises a heating stage under steam heating and a boiling holding stage under steam heating; the boiling holding stage is conducted with a heat preservation time of 40 minutes to 90 minutes, and the boiling holding stage is conducted under a steam pressure of lower than or equal to 0.1 MPa; and the heating stage is conducted under a steam pressure of lower than or equal to 0.2 MPa.

3. The method for preparing the Zhu Ling Tang extract solution of claim 1, wherein during the second boiling extraction, the ratio of the mass of the water to the total mass of the four traditional Chinese medicines is in a range of 6:1 to 12:1; and

the second boiling extraction comprises a heating stage under steam heating and a boiling holding stage under steam heating; the boiling holding stage is conducted with a heat preservation time of 25 minutes to 60 minutes, and the boiling holding stage is conducted under a steam pressure of lower than or equal to 0.1 MPa; and the heating stage is conducted under a steam pressure of lower than or equal to 0.2 MPa.

4. The method for preparing the Zhu Ling Tang extract solution of claim 1, wherein a mass ratio of the *Polyporus umbellatus,* the *Poria cocos,* the *Rhizoma Alismatis,* and the *Talcum* is in a range of (0.7-1.3):(0.7-1.3):(0.7-1.3):(0.7-1.3).

5. The method for preparing the Zhu Ling Tang extract solution of claim 1, wherein the first concentration and the second concentration each are conducted independently under steam heating; and

the first concentration and the second concentration each are conducted at a temperature independently of 70°C to 90°C under a vacuum pressure independently of 0.03 MPa to 0.06 MPa; the first concentration and the second concentration each are conducted with a heat preservation time independently of less than or equal to 6 hours; and the first concentration and the second concentration each are conducted under a steam pressure independently of 0.01 MPa to 0.05 MPa.

6. The method for preparing the Zhu Ling Tang extract solution of claim 1, wherein in the aqueous solution of the *Asini Corii Colla,* a mass ratio of the *Asini Corii Colla* to water is in a range of 1:2 to 1:6.

7. A method for preparing a Zhu Ling Tang extract paste, comprising:
concentrating the Zhu Ling Tang extract solution prepared by the method for preparing the Zhu Ling Tang extract solution of any one of claims 1 to 6 to obtain the Zhu Ling Tang extract paste, wherein the concentrating is conducted at a temperature of lower than or equal to 90°C; and a relative density of the Zhu Ling Tang extract paste at 20°C is in a range of 1.12 to 1.16.

8. The method for preparing the Zhu Ling Tang extract paste of claim 7, wherein the concentrating is conducted under steam heating; and

the concentrating is conducted at a temperature of 70°C to 90°C under a vacuum pressure of 0.03 MPa to 0.06 MPa; the concentrating is conducted with a heat preservation time of less than or equal to 6 hours; and the concentrating is conducted under a steam pressure of 0.01 MPa to 0.05 MPa.

9. A method for preparing a Zhu Ling Tang solid preparation, the Zhu Ling Tang solid preparation comprising a Zhu Ling Tang dry powder or a Zhu Ling Tang granule; wherein

under a condition that the Zhu Ling Tang solid preparation is the Zhu Ling Tang dry powder, the method comprises:
mixing the Zhu Ling Tang extract solution prepared by the method for preparing the Zhu Ling Tang extract solution of any one of claims 1 to 6 with an aqueous solution of an adjuvant, and concentrating a resulting mixture to obtain a mixed thick paste; and vacuum-drying the mixed thick paste to obtain the Zhu Ling Tang dry powder, wherein a mass ratio of the adjuvant to the Zhu Ling Tang extract solution is in a range of 1:50 to 7:50, and a mass of the Zhu Ling Tang extract solution is based on a total mass of the *Polyporus umbellatus,* the *Poria cocos,* the *Rhizoma Alismatis,* the *Talcum,* and the *Asini Corii Colla;* and
under a condition that the Zhu Ling Tang solid preparation is the Zhu Ling Tang granule, the method comprises:
conducting dry granulation on the Zhu Ling Tang dry powder to obtain the Zhu Ling Tang granule.

10. The method for preparing the Zhu Ling Tang solid preparation of claim 9, wherein the adjuvant is at least one selected from the group consisting of dextrin, lactose, and soluble starch.

11. The method for preparing the Zhu Ling Tang solid preparation of claim 9, wherein the concentrating is conducted under steam heating at a temperature of 70°C to 90°C under a vacuum pressure of 0.03 MPa to 0.06 MPa; the concentrating is conducted with a heat preservation time of less than or equal to 6 hours; and the concentrating is conducted under a heating steam pressure of 0.01 MPa to 0.05 MPa.

12. The method for preparing the Zhu Ling Tang solid preparation of claim 9 or 11, wherein a relative density of the mixed thick paste at 20°C is in a range of 1.12 to 1.16.

13. The method for preparing the Zhu Ling Tang solid preparation of claim 9, wherein the vacuum-drying is conducted by vacuum belt drying, and working parameters for the vacuum belt drying comprise: a feeding temperature: 75°C to 85°C; a feeding rate: 2.0 L/h to 3.5 L/h; a running speed of a track: 150±50 mm/min; a rotational speed of a material-distributing motor: 100 r/min; an angle of a swingarm: 12° to 25°; heating temperatures: 95±5°C in a first zone, 96±5°C in a second zone, and 96±5°C in a third zone; a vacuum degree: 0.097 MP to 0.1 MP; a speed of a cutter: 10 seconds/time to 20 seconds/time; and a cooling temperature: 30±5°C; and

the dry granulation is conducted in a granulator, and working parameters for the dry granulation comprise: a pressure of a press roller of the granulator: 12 MPa to 20 MPa; a rotational speed of the press roller of the granulator: 4 r/min to 10 r/min; a vertical rotational speed of a screw feeder of the granulator: 25 r/min to 40 r/min; a horizontal rotational speed of the screw feeder of the granulator: 70 r/min to 120 r/min; and a mesh size of a sieve: 14 mesh.

14. A Zhu Ling Tang solid preparation prepared by the method for preparing the Zhu Ling Tang solid preparation of any one of claims 9 to 13, wherein a dry extract yield of the Zhu Ling Tang solid preparation is in a range of 18% to 23%; a total content of alisol B 23-acetate and alisol C 23-acetate in the Zhu Ling Tang solid preparation is in a range of 0.011 wt% to 0.020 wt%; and a total content of a donkey-derived polypeptide $A_1$ and a donkey-derived polypeptide $A_2$ in the Zhu Ling Tang solid preparation is in a range of 0.091 wt% to 0.18 wt%.

**FIG. 1**

**FIG. 2**

## INTERNATIONAL SEARCH REPORT

| International application No. |
|---|
| **PCT/CN2023/104721** |

### A. CLASSIFICATION OF SUBJECT MATTER

A61K9/00(2006.01)i; A61K36/884(2006.01)i; A61K9/14(2006.01)i; A61K9/16(2006.01)i

According to International Patent Classification (IPC) or to both national classification and IPC

### B. FIELDS SEARCHED

Minimum documentation searched (classification system followed by classification symbols)

IPC:A61K9; A61K36; A61K35; A61J3

Documentation searched other than minimum documentation to the extent that such documents are included in the fields searched

Electronic data base consulted during the international search (name of data base and, where practicable, search terms used)

CJFD, CNTXT, ENTXT, ENTXTC, VEN, WPABSC, PUBMED, ELSEVIER, 必应, BING, CNKI, 万方, WANFANG, 百度, BAIDU, 超星读秀, DUXIU: 猪苓, 茯苓, 泽泻, 滑石, 阿胶, 浸泡, 提取, 沸腾, 煮沸, 浓缩, 保温, 干燥, 第二, 粉剂, 颗粒剂, granules, powder, polyporus umbellatus, UmbeLlate Pore Decoction, zhuling decoction

### C. DOCUMENTS CONSIDERED TO BE RELEVANT

| Category* | Citation of document, with indication, where appropriate, of the relevant passages | Relevant to claim No. |
|---|---|---|
| PX | CN 115554228 A (DONG-E-E-JIAO CO., LTD.) 03 January 2023 (2023-01-03) description, paragraphs 37-109 | 1-14 |
| Y | CN 110946761 A (DONG-E-E-JIAO CO., LTD.) 03 April 2020 (2020-04-03) embodiments | 1-14 |
| Y | 刘洁 等 (LIU, Jie et al.). "茯苓配方颗粒、猪苓配方颗粒标准汤剂的质量评价 (Quality Evaluation for Standard Decoction of Fuling Formula Granules and Zhuling Formula Granules)" 中成药 (Chinese Traditional Patent Medicine), Vol. 42, No. 8, 31 August 2020 (2020-08-31), 2003-2008 ISSN: 1001-1528, page 2003, right column, paragraph 2, section 2.1 | 1-14 |
| Y | CN 102846877 A (SUZHOU ZHIWEITANG BIOTECHNOLOGY CO., LTD. et al.) 02 January 2013 (2013-01-02) description, paragraphs 27-38 | 1-14 |

✓ Further documents are listed in the continuation of Box C.　　✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **13 September 2023** | **28 September 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2023/104721** |

### C. DOCUMENTS CONSIDERED TO BE RELEVANT

| Category* | Citation of document, with indication, where appropriate, of the relevant passages | Relevant to claim No. |
|---|---|---|
| Y | CN 102283954 A (SUZHOU ZHIWEITANG BIOTECHNOLOGY CO., LTD. et al.) 21 December 2011 (2011-12-21)<br>description, paragraphs 17-30 | 1-14 |
| A | JP H07309769 A (INDUSTRIAL TECHNOLOGY RESEARCH INSTITUTE) 28 November 1995 (1995-11-28)<br>embodiments | 1-14 |
| A | JP 6918393 B1 (OMINEDO YAKUHIN KOGYO KK) 11 August 2021 (2021-08-11)<br>description, paragraphs 6-36 | 1-14 |

Form PCT/ISA/210 (second sheet) (July 2022)

### INTERNATIONAL SEARCH REPORT
**Information on patent family members**

International application No.

**PCT/CN2023/104721**

| Patent document cited in search report | | | Publication date (day/month/year) | Patent family member(s) | | | Publication date (day/month/year) |
|---|---|---|---|---|---|---|---|
| CN | 115554228 | A | 03 January 2023 | None | | | |
| CN | 110946761 | A | 03 April 2020 | None | | | |
| CN | 102846877 | A | 02 January 2013 | None | | | |
| CN | 102283954 | A | 21 December 2011 | None | | | |
| JP | H07309769 | A | 28 November 1995 | JP | 2656727 | B2 | 24 September 1997 |
| JP | 6918393 | B1 | 11 August 2021 | JP | 2022130003 | A | 06 September 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211174012

**Non-patent literature cited in the description**

- Chinese Pharmacopoeia. 2020

- **ZHU LING TANG**. Chinese Pharmacopoeia. 2020